# EUROPEAN PATENT SPECIFICATION

(11) **EP 2 276 481 B1**
(45) Date of publication and mention of the grant of the patent: **30.07.2014**
(21) Application number: 08732830.8
(22) Date of filing: 26.03.2008
(51) Int. Cl.: A61K 31/353, A61K 31/357, A61P 25/22

(54) **USE OF BENZO-FUSED HETEROCYCLE SULFAMIDE DERIVATIVES FOR THE TREATMENT OF ANXIETY**
VERWENDUNG VON BENZOKONDENSIERTEN HETEROCYCLISCHEN SULFAMIDDERIVATEN ZUR BEHANDLUNG VON ANGSTZUSTÄNDEN
UTILISATION DE DÉRIVÉS DE SULFAMIDE HÉTÉROCYCLIQUE BENZO-CONDENSÉ POUR LE TRAITEMENT DE L ANXIÉTÉ

(43) Date of publication of application: 26.01.2011
(73) Proprietor: Janssen Pharmaceutica, N.V., 2340 Beerse (BE)
(72) Inventor: SMITH-SWINTOSKY, Virginia L., Hatfield, Pennsylvania 19440 (US)
(74) Representative: Warner, James Alexander
(86) International application number: PCT/US2008/058210
(87) International publication number: WO 2009/120189

(56) References cited:
- WO-A-2007/075717
- WO-A-2007/075751
- WO-A-2007/075834
- MULA MARCO ET AL: "The role of anticonvulsant drugs in anxiety disorders: a critical review of the evidence" JOURNAL OF CLINICAL PSYCHOPHARMACOLOGY, WILLIAMS AND WILKINS, vol. 27, no. 3, 1 January 2007 (2007-01-01), pages 263-272, XP009108472 ISSN: 0271-0749
- SCOZZAFAVA A ET AL: "Modulation of carbonic anhydrase activity and its applications in therapy" EXPERT OPINION ON THERAPEUTIC PATENTS, INFORMA HEALTHCARE, GB, vol. 14, no. 5, 1 January 2004 (2004-01-01), pages 667-702, XP002431413 ISSN: 1354-3776
- MARYANOFF B E ET AL: "Comparison of Sulfamate and Sulfamide Groups for the Inhibition of Carbonic Anhydrase-II by Using Topiramate as a Structural Platform" JOURNAL OF MEDICINAL CHEMISTRY, US AMERICAN CHEMICAL SOCIETY. WASHINGTON, vol. 48, no. 6, 13 December 2004 (2004-12-13), pages 1941-1947, XP002345002 ISSN: 0022-2623

## Description

### FIELD OF THE INVENTION

The present invention is directed to the use of benzo-fused heterocycle sulfamide derivatives for the treatment of anxiety and related disorders including generalized anxiety disorder, acute stress disorder, post traumatic stress disorder, obsessive-compulsive disorder, social phobia, specific phobia, panic disorder with or without agoraphobia, agoraphobia without a history of panic disorder, anxiety disorder due to general medical condition, substance abuse induced anxiety disorder and anxiety disorder not otherwise specified.

### BACKGROUND OF THE INVENTION

Anxiety disorders, the most prevalent psychiatric illnesses in the general community, are present in 15 to 20% of medical clinic patients. Anxiety, defined as a subjective sense of unease, dread, or foreboding, can indicate a primary psychiatric condition or can be a component of, or reaction to, a primary medical disease. The primary anxiety disorders are classified according to their duration and course and the existence and nature of precipitants.

Patients with **generalized anxiety disorder (GAD)** have persistent, excessive, and/or unrealistic worry associated with muscle tension, impaired concentration, autonomic arousal, feeling "on edge" or restless, and insomnia. Onset is usually before age 20, and a history of childhood fears and social inhibition may be present. The lifetime prevalence of GAD is 5 to 6%; the risk is higher in first-degree relatives of patients with the diagnosis. Interestingly, family studies indicate that GAD and panic disorder segregate independently. Over 80% of patients with GAD also suffer from major anxiety and related disorders, dysthymia, or social phobia. Comorbid substance abuse is common in these patients, particularly alcohol and/or sedative/hypnotic abuse. Patients with GAD worry excessively over minor matters, with life-disrupting effects; unlike in panic disorder, complaints of shortness of breath, palpitations, and tachycardia are relatively rare.

A combination of pharmacologic and psychotherapeutic interventions is most effective in the treatment of anxiety, but complete symptomatic relief is rare. A short course of a benzodiazepine is usually indicated, preferably lorezapam, oxazepam or temazepam. Administration is generally initiated at the lowest dose possible and prescribed on an as-needed basis as symptoms warrant. Benzodiazepines differ in their milligram per kilogram potency, half-life, lipid solubility, metabolic pathways, and presence of active metabolites. Agents that are absorbed rapidly and are lipid soluble, such as diazepam, have a rapid onset of action and a higher abuse potential. Benzodiazepines are generally not be prescribed for >4 to 6 weeks because of the development of tolerance and the risk of abuse and dependence. It is also necessary to warn patients that concomitant use of alcohol or other sedating drugs may be neurotoxic and impair their ability to function.

Adverse effects of benzodiazepines generally parallel their relative half-lives. Longer-acting agents, such as diazepam, chlordiazepoxide. flurazepam and clonazepam, tend to accumulate active metabolites, with resultant sedation, impairment of cognition, and poor psychomotor performance. Shorter-acting compounds, such as alprazolam and oxazepam, can produce daytime anxiety, early morning insomnia, and, with discontinuation, rebound anxiety and insomnia. Although patients develop tolerance to the sedative effects of benzodiazepines, they are less likely to habituate to the adverse psychomotor effects. Withdrawal from the longer half-life benzodiazepines can be accomplished through gradual, stepwise dose reduction (by 10% every 1 to 2 weeks) over 6 to 12 weeks. It is usually more difficult to taper patients off shorter-acting benzodiazepines. Physicians may need to switch a patient to a benzodiazepine with a longer half-life or use an adjunctive medication, such as a beta blocker or carbamazepine, before attempting to discontinue the benzodiazepine. Withdrawal reactions vary in severity and duration; they can include anxiety and related disorders, anxiety, delirium, lethargy, diaphoresis, tinnitus, autonomic arousal, adventitious movements, and, rarely, seizures.

An alternative treatment is buspirone. Buspirone is a nonbenzodiazepine anxiolytic agent. It is nonsedating, does not produce tolerance or dependence, does not interact with benzodiazepine receptors or alcohol, and has no abuse or disinhibition potential. However, it requires several weeks to take effect and requires thrice-daily dosing. Patients who were previously responsive to a benzodiazepine are unlikely to rate buspirone as equally effective, but patients with head injury or dementia who have symptoms of anxiety and/or agitation may do well with this agent.

Administration of benzodiazepines to geriatric patients requires special care. Such patients have increased drug absorption; decreased hepatic metabolism, protein binding, and renal excretion; and an increased volume of distribution. These factors, together with the likely presence of comorbid medical illnesses and medication, dramatically increase the likelihood of toxicity. Iatrogenic psychomotor impairment can result in falls and fractures, confusional states, or motor vehicle accidents. If used, agents in this class should be started at the lowest possible dose, and effects should be monitored closely. Benzodiazepines are also contraindicated during pregnancy and breast-feeding.

Anticonvulsants with GABAergic properties may also be effective against anxiety. Gabapentic, oxcarbazepine, tiagabine, pregabalin and divalproex have all shown some degree of benefit in a variety of anxiety-related syndromes. Agents that selectively target GABA_{A} receptor subtypes are currently under development; and it is hoped that these will lack the sedating, memory-impairing, and addicting properties of benzodiazepines.

Panic disorder is defined by the presence of recurrent and unpredictable panic attacks, which are distinct episodes of intense fear and discomfort associated with a variety of physical symptoms, including palpitations, sweating, trembling, shortness of breath, chest pain, dizziness, and a fear of impending doom or death. Paresthesias, gastrointestinal distress, and feelings of unreality are also common. Diagnostic criteria also require at least 1 month of concern or worry about the attacks or a change in behavior related to them. The lifetime prevalence of panic disorder is 1 to 3%. Panic attacks have a sudden onset, developing within 10 min and usually resolving over the course of an hour, and they occur in an unexpected fashion. The frequency and severity of panic attacks vary, ranging from once a week to clusters of attacks separated by months of well-being. The first attack is usually outside the home, and onset is typically in late adolescence to early adulthood. In some individuals, anticipatory anxiety develops over time and results in a generalized fear and a progressive avoidance of places or situations in which a panic attack might recur. *Agoraphobia,* which occurs commonly in patients with panic disorder, is an acquired irrational fear of being in places where one might feel trapped or unable to escape. Typically, it leads the patient into a progressive restriction in lifestyle and, in a literal sense, in geography. Frequently, patients are embarrassed that they are housebound and dependent on the company of others to go out into the world and do not volunteer this information; thus physicians will fail to recognize the syndrome if direct questioning is not pursued.

Achievable goals of treatment are to decrease the frequency of panic attacks and to reduce their intensity. The cornerstone of drug therapy is antidepressant medication. The tricyclic antidepressants (TCAs) imipramine and clomipramine benefit 75 to 90% of panic disorder patients. Low doses (e.g., 10 to 25 mg/d) are given initially to avoid transient increased anxiety associated with heightened monoamine levels. Selective serotonin reuptake inhibitors (SSRIs) are equally effective and do not have the adverse effects of TCAs. SSRIs should be started at one-third to one-half of their usual antidepressant dose (e.g., 5 to 10 mg fluoxetine, 25 to 50 mg sertraline, 10 mg paroxetine). Monoamine oxidase inhibitors (MAOIs) are also effective and may specifically benefit patients who have comorbid features of atypical depression (i.e., hypersomnia and weight gain). Insomnia, orthostatic hypotension, and the need to maintain a low-tyramine diet (avoidance of cheese and wine) have limited their use, however. Antidepressants typically take 2 to 6 weeks to become effective, and doses may need to be adjusted based upon the clinical response.

The cardinal feature of phobic disorders is a marked and persistent fear of objects or situations, exposure to which results in an immediate anxiety reaction. The patient avoids the phobic stimulus, and this avoidance usually impairs occupational or social functioning. Panic attacks may be triggered by the phobic stimulus or may occur spontaneously. Unlike patients with other anxiety disorders, individuals with phobias usually experience anxiety only in specific situations. Common phobias include fear of closed spaces (claustrophobia), fear of blood, and fear of flying. **Social phobia, also known as social anxiety disorder,** is distinguished by a specific fear of social or performance situations in which the individual is exposed to unfamiliar individuals or to possible examination and evaluation by others. Examples include having to converse at a party, use public restrooms, and meet strangers. In each case, the affected individual is aware that the experienced fear is excessive and unreasonable given the circumstance. The specific content of a phobia may vary across gender, ethnic, and cultural boundaries. Phobic disorders are common, affecting ∼10% of the population.

MAOIs alleviate social phobia independently of their antidepressant activity, and SSRIs appear to be effective also. Benzodiazepines can be helpful in reducing fearful avoidance, but the chronic nature of phobic disorders limits their usefulness.

Patients may also develop **anxiety after exposure to extreme traumatic events** such as the threat of personal death or injury or the death of a loved one. The reaction may occur shortly after the trauma *(acute stress disorder)* or be delayed and subject to recurrence (PTSD). In both syndromes, individuals experience associated symptoms of detachment and loss of emotional responsivity. The patient may feel depersonalized and unable to recall specific aspects of the trauma, though typically it is re-experienced through intrusions in thought, dreams, or flashbacks, particularly when cues of the original event are present. Patients often actively avoid stimuli that precipitate recollections of the trauma and demonstrate a resulting increase in vigilance, arousal, and startle response. Patients with stress disorders are at risk for the development of other disorders related to anxiety, mood, and substance abuse (especially alcohol). Between 5 and 10% of Americans will at some time in their life satisfy criteria for PTSD, with women more likely to be affected than men. Risk factors for the development of PTSD include a past psychiatric history and personality characteristics of high neuroticism and extroversion.

Acute stress reactions are usually self-limited, and treatment typically involves the short-term use of benzodiazepines and supportive/expressive psychotherapy. The chronic and recurrent nature of PTSD, however, requires a more complex approach employing drug and behavioral treatments. PTSD is highly correlated with peritraumatic dissociative symptoms and the development of an acute stress disorder at the time of the trauma. TCAs such as imipramine and amytriptyline, the MAOI phenelzine, and the SSRIs (fluoxetine, sertraline, citalopram, paroxetine) can all reduce anxiety, symptoms of intrusion, and avoidance behaviors, as can prazosin, an α₁ antagonist. Trazodone, a sedating antidepressant, is frequently used at night to help with insomnia (50 to 150 mg qhs). Carbamazepine, valproic acid and alprazolam have also independently produced improvement in uncontrolled trials.

**Obsessive-compulsive disorder (OCD)** is characterized by obsessive thoughts and compulsive behaviors that impair everyday functioning. Fears of contamination and germs are common, as are handwashing, counting behaviors, and having to check and recheck such actions as whether a door is locked. The degree to which the disorder is disruptive for the individual varies, but in all cases obsessive-compulsive activities take up >1 h/d and are undertaken to relieve the anxiety triggered by the core fear. Patients often conceal their symptoms, usually because they are embarrassed by the content of their thoughts or the nature of their actions. Physicians must ask specific questions regarding recurrent thoughts and behaviors, particularly if physical clues such as chafed and reddened hands or patchy hair loss (from repetitive hair pulling, or trichotillomania) are present. Comorbid conditions are common, the most frequent being anxiety and related disorders, other anxiety disorders, eating disorders, and tics. OCD has a lifetime prevalence of 2 to 3% worldwide. Onset is usually gradual, beginning in early adulthood, but childhood onset is not rare. The disorder usually has a waxing and waning course, but some cases may show a steady deterioration in psychosocial functioning.

Clomipramine, fluoxetine and fluvoxamine are approved for the treatment of OCD. Clomipramine is a TCA that is often tolerated poorly owing to anticholinergic and sedative side effects at the doses required to treat the illness (150 to 250 mg/d). Its efficacy in OCD is unrelated to its antidepressant activity. Fluoxetine (40 to 60 mg/d) and fluvoxamine (100 to 300 mg/d) are as effective as clomipramine and have a more benign side-effect profile. Only 50 to 60% of patients with OCD show adequate improvement with pharmacotherapy alone. In treatment-resistant cases, augmentation with other serotonergic agents, such as buspirone, or with a neuroleptic or benzodiazepine may be beneficial. When a therapeutic response is achieved, long-duration maintenance therapy is usually indicated. Additionally, for many individuals, particularly those with time-consuming compulsions, behavior therapy will result in as much improvement as that afforded by medication. Effective techniques include the gradual increase in exposure to stressful situations, maintenance of a diary to clarify stressors, and homework assignments that substitute new activities for compulsive behaviors.

(Harrison's Online @ www.accessmedicine.com, October 10, 2006)

WO2007/075751 discloses the use of the presently claimed compounds in the treatment of depression.

WO2007/075717 discloses the use of the presently claimed compounds in the treatment of substance abuse and addiction.

Nonetheless, there remains a need to provide an effective treatment for anxiety and related disorders.

### SUMMARY OF THE INVENTION

The present invention is directed to a method for the treatment of anxiety and related disorders comprising administering to a subject in need thereof a therapeutically effective amount of a compound of formula (I) 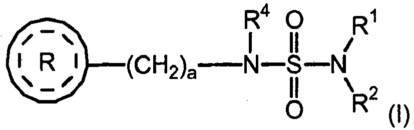 wherein
R¹ and R² are each independently selected from the group consisting of hydrogen and lower alkyl;
R⁴ is selected from the group consisting of hydrogen and lower alkyl;
a is an integer from 1 to 2; 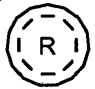 is selected from the group consisting of 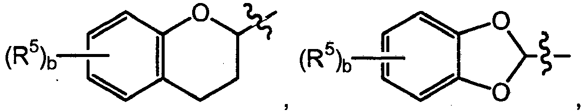 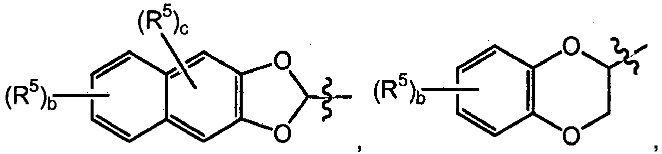 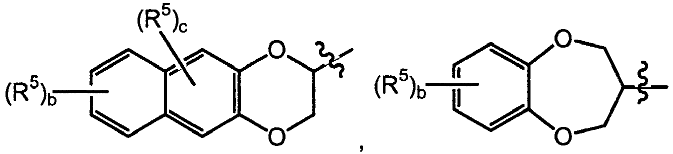 and 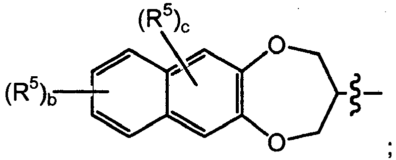
wherein b is an integer from 0 to 4; and wherein c is an integer from 0 to 2;
each R⁵ is independently selected from the group consisting of halogen, and lower alkyl;
provided that when 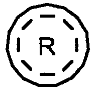 is 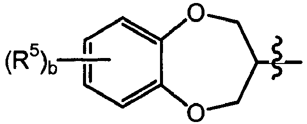 or 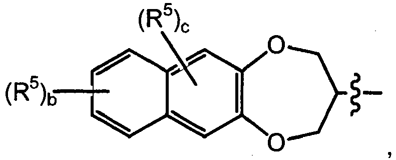 then a is 1;
or a pharmaceutically acceptable salt thereof.

The present invention is further directed to a method for the treatment of anxiety and related disorders comprising administering to a subject in need thereof a therapeutically effective amount of compound of formula (II) 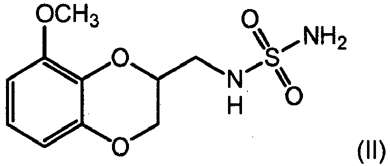 or a pharmaceutically acceptable salt thereof.

The present invention is further directed to a method for the treatment of anxiety and related disorders comprising administering to a subject in need thereof co-therapy with a therapeutically effective amount of at least one anxiolytic agent and a compound of formula (I) or formula (II) as herein defined.

Exemplifying the invention are methods of treating generalized anxiety disorder, acute stress disorder, post traumatic stress disorder, obsessive-compulsive disorder, social phobia, specific phobia, panic disorder with or without agoraphobia, agoraphobia without a history of panic disorder, and substance abuse induced anxiety disorder comprising administering to a subject in need thereof a therapeutically effective amount of any of the compounds or pharmaceutical compositions described above.

### DETAILED DESCRIPTION OF THE INVENTION

The present invention relates to the treatment of anxiety and related disorders comprising administering to a subject in need thereof a therapeutically effective amount of a compound of formula (I) 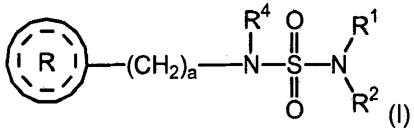 or a pharmaceutically acceptable salt thereof, wherein 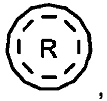 a, R¹, R² and R⁴ are as herein defined. The present invention is further directed to the treatment of anxiety and related disorders comprising administering to a subject in need thereof a therapeutically effective amount of a compound of formula (I) or formula (II) in combination with at least one anxiolytic.

In an embodiment of the present invention, the compound of formula (I) is Compound #8 or a pharmaceutically acceptable salt thereof, a compound of the following structure 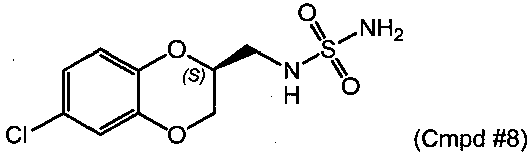 also known as (2S)-(-)-N-(6-chloro-2,3-dihydro-benzo[1,4]dioxin-2-ylmethyl)-sulfamide.

As used herein, the terms **"anxiety and related disorders"** and **"anxiety or a related disorder"** shall be defined to include anxiety and related disorders including generalized anxiety disorder, acute stress disorder, post traumatic stress disorder, obsessive-compulsive disorder, social phobia (also known as social anxiety disorder), specific phobia, panic disorder with or without agoraphobia, agoraphobia without a history of panic disorder, anxiety disorder due to general medical condition, substance abuse induced anxiety disorder and anxiety disorder not otherwise specified (as these conditions are described by their diagnostic criteria, as listed in the Diagnostic and Statistical Manual of Mental Disorders, 4th Edition, Text Revision, American Psychiatric Association, 2000, incorporated herein by reference). Preferably, the anxiety or related disorder is selected from the group consisting of generalized anxiety disorder, acute stress disorder, post traumatic stress disorder and obsessive-compulsive disorder. More preferably, the anxiety and related disorder is generalized anxiety disorder.

As used herein, unless otherwise noted, the term **"anxiolytic"** shall mean any pharmaceutical agent, which may be used to treat anxiety or a related disorders, or the symptoms of anxiety or a related disorder. The term "anxiolytic" further includes, but is not limited to, any pharmaceutical agent approved by the FDA or other regulatory agency for the treatment of anxiety or a related disorder. One skilled in the art may readily identify such pharmaceutical agents by consulting the web site(s), pharmaceutical references (such as the Physician's Desk Reference) and / or publications of the FDA or other regulatory agency. Further, one skilled in the art would be able to readily determined recommended dosage levels for known and / or marketed anxiolytic drugs by consulting appropriate references such as drug package inserts, FDA guidelines, the Physician's Desk Reference, and the like.

Suitable examples of anxiolytics include, but are not limited to benzodiazepines, selective serotonin reuptake inhibitors (SSRIs), selective noradrenergic reuptake inhibitors, serotonin receptor antagonists, norepinephrine reuptake inhibitors, dopamine reuptake inhibitors, dopamine receptor antagonist (preferably, antagonists of the D1, D2 and / or D3 dopamine receptor), corticotropin releasing factor (CRF) antagonists, monoamine oxidase (MAO) inhibitors, 5HT₁ₐ agonists, Alpha2 adrenergic antagonists, GABA_{A} agonists, GABA_{B} antagonists, cannabanoid agonists (preferably, CB1 receptor agonists), neurokinin antagonists (preferably, NK1 receptor antagonists), kappa opiod antagonists, Opiod Receptor Like-1 (ORL-1) agonists (also known as Nociceptin Orphanin FQ Peptide (NOP) agonists), and the like. Preferably, the anxiolytic is selected from the group consisting of benzodiazepines, selective serotonin reuptake inhibitors (SSRIs) and selective noradrenergic reuptake inhibitors.

In an embodiment of the present invention, the anxiolytic is selected from the group consisting of benzodiazepines such as diazepam, flurazepam, triazolam, lorazepam, alprazolam, chlordiazepam, oxazepam, temazepam and clonazepam; non-benzodiazepine such as buspirone; selective serotonin reuptake inhibitors such as fluoxetine, sertraline, paroxetine, citalopram, fluvoxamine, and the like; serotonin receptor antagonists such as nefazadone, and the like; serotonin noradrenergic reuptake inhibitors such as venlafaxine, milnacipran and the like, 3-(3-amino-2-(R)-hydroxy-propyl)-1-(4-fluoro-phenyl)-8-(8-methyl-naphthalen-1-ylmethyl)-1,3,8-triazaspiro[4.5]decan-4-one, 2-(2-chloro-phenyl)-2-(S)-hydroxy-ethyl ester carbamic acid (also known as carisbamate) and 1-[7-(4-bromo-2,6-dimethylphenyl)-2,5-dimethyl-7H-pyrrolo[2,3-d]pyrimidin-4-yl]-4-piperidinemethanol. Preferably, the anxiolytic is selected from the group consisting of diazepam, venlafaxine, fluoxetine and pregabalin.

The term **"subject"** as used herein, refers to an animal, preferably a mammal (e.g. domesticated animals such as dogs, cats, etc.), most preferably a human, who has been the object of treatment, observation or experiment.

The term **"therapeutically effective amount"** as used herein, means that amount of active compound or pharmaceutical agent that elicits the biological or medicinal response in a tissue system, animal or human that is being sought by a researcher, veterinarian, medical doctor or other clinician, which includes alleviation of the symptoms of the disease or disorder being treated.

Wherein the present invention is directed to co-therapy or combination therapy, comprising administration of one or more compound(s) of formula (I) or formula (II) and one or more anxiolytics, "therapeutically effective amount" shall mean that amount of the combination of agents taken together so that the combined effect elicits the desired biological or medicinal response. For example, the therapeutically effective amount of co-therapy comprising administration of a compound of formula (I) or formula (II) and at least on anxiolytic would be the amount of the compound of formula (I) or formula (II) and the amount of the anxiolytic that when taken together or sequentially have a combined effect that is therapeutically effective. Further, it will be recognized by one skilled in the art that in the case of co-therapy with a therapeutically effective amount, as in the example above, the amount of the compound of formula (I) or formula (II) and/or the amount of the anxiolytic individually may or may not be therapeutically effective.

As used herein, the terms **"co-therapy"** and **"combination therapy"** shall mean treatment of a subject in need thereof by administering one or more compounds of formula (I) or formula (II) in combination with one or more anxiolytic(s), wherein the compound(s) of formula (I) or formula (II) and the anxiolytic(s) are administered by any suitable means, simultaneously, sequentially, separately or in a single pharmaceutical formulation. Where the compound(s) of formula (I) or formula (II) and the anxiolytic(s) are administered in separate dosage forms, the number of dosages administered per day for each compound may be the same or different. The compound(s) of formula (I) or formula (II) and the anxiolytic(s) may be administered via the same or different routes of administration. Examples of suitable methods of administration include, but are not limited to, oral, intravenous (iv), intramuscular (im), subcutaneous (sc), transdermal, and rectal. Compounds may also be administered directly to the nervous system including, but not limited to, intracerebral, intraventricular, intracerebroventricular, intrathecal, intracisternal, intraspinal and / or peri-spinal routes of administration by delivery via intracranial or intravertebral needles and / or catheters with or without pump devices. The compound(s) of formula (I) or formula (II) and the anxiolytic(s) may be administered according to simultaneous or alternating regimens, at the same or different times during the course of the therapy, concurrently in divided or single forms.

In an embodiment, the present invention relates to the treatment of anxiety and related disorders comprising administering to a subject in need thereof co-therapy comprising one or more compounds of formula (I) or formula (II) and one or more anxiolytic compounds selected from the group consisting of benzodiazepines, selective serotonin reuptake inhibitors (SSRIs) and selective noradrenergic reuptake inhibitors.

In another embodiment, the present invention relates to the treatment of anxiety and related disorders comprising administering to a subject in need thereof a co-therapy comprising one or more compounds of formula (I) or formula (II) and one or more anxiolytics selected from the group consisting of benzodiazepines such as diazepam, flurazepam, triazolam, lorazepam, alprazolam, chlordiazepam, oxazepam, temazepam and clonazepam; non-benzodiazepine such as buspirone; selective serotonin reuptake inhibitors such as fluoxetine, sertraline, paroxetine, citalopram, fluvoxamine, and the like; serotonin receptor antagonists such as nefazadone, and the like; serotonin noradrenergic reuptake inhibitors such as venlafaxine, milnacipran and the like, 3-(3-amino-2-(R)-hydroxy-propyl)-1-(4-fluoro-phenyl)-8-(8-methyl-naphthalen-1-ylmethyl)-1,3,8-triazaspiro[4.5]decan-4-one, 2-(2-chloro-phenyl)-2-(S)-hydroxy-ethyl ester carbamic acid (also known as carisbamate) and 1-[7-(4-bromo-2,6-dimethylphenyl)-2,5-dimethyl-7H-pyrrolo[2,3-d]pyrimidin-4-yl]-4-piperidinemethanol.

Preferably, one or more compounds of formula (I) or formula (II) are administered as co-therapy with one or more anxiolytics selected from the group consisting of diazepam, venlafaxine, fluoxetine and pregabalin.

In an embodiment, the present invention relates to the treatment of anxiety and related disorders comprising administering to a subject in need thereof co-therapy comprising Compound #8 or a pharmaceutically acceptable salt thereof and one or more anxiolytic compounds selected from the group consisting of benzodiazepines, selective serotonin reuptake inhibitors (SSRIs) and selective noradrenergic reuptake inhibitors.

In another embodiment, the present invention relates to the treatment of anxiety and related disorders comprising administering to a subject in need thereof a co-therapy comprising Compound #8 or a pharmaceutically acceptable salt thereof and one or more anxiolytics selected from the group consisting of benzodiazepines such as diazepam, flurazepam, triazolam, lorazepam, alprazolam, chlordiazepam, oxazepam, temazepam and clonazepam; non-benzodiazepine such as buspirone; selective serotonin reuptake inhibitors such as fluoxetine, sertraline, paroxetine, citalopram, fluvoxamine, and the like; serotonin receptor antagonists such as nefazadone, and the like; serotonin noradrenergic reuptake inhibitors such as venlafaxine, milnacipran and the like, 3-(3-amino-2-(R)-hydroxy-propyl)-1-(4-fluoro-phenyl)-8-(8-methyl-naphthalen-1-ylmethyl)-1,3,8-triazaspiro[4.5]decan-4-one, 2-(2-chloro-phenyl)-2-(S)-hydroxy-ethyl ester carbamic acid (also known as carisbamate) and 1-[7-(4-bromo-2,6-dimethylphenyl)-2,5-dimethyl-7H-pyrrolo[2,3-d]pyrimidin-4-yl]-4-piperidinemethanol.

Preferably, Compound #8 or a pharmaceutically acceptable salt thereof is administered as co-therapy with one or more anxiolytics selected from the group consisting of diazepam, venlafaxine, fluoxetine and pregabalin.

In an embodiment of the present invention R¹ is selected from the group consisting of hydrogen and methyl. In another embodiment of the present invention R² is selected from the group consisting of hydrogen and methyl. In yet another embodiment of the present invention R¹ and R² are each hydrogen or R¹ and R² are each methyl.

In an embodiment of the present invention -(CH₂)ₐ- is selected from the group consisting of -CH₂- and -CH₂-CH₂-. In another embodiment of the present invention -(CH₂)ₐ- is -CH₂-.

In an embodiment of the present R⁴ is selected from the group consisting of hydrogen and methyl, preferably, R⁴ is hydrogen.

In an embodiment of the present invention a is 1.

In an embodiment of the present invention b is an integer from 0 to 2. In another embodiment of the present invention c is an integer from 0 to 2. In another embodiment of the present invention b is an integer from 0 to 1. In another embodiment of the present invention c is an integer from 0 to 1. In yet another embodiment of the present invention the sum of b and c is an integer form 0 to 2, preferably an integer form 0 to 1. In yet another embodiment of the present invention b is an integer from 0 to 2 and c is 0.

In an embodiment of the present invention, 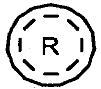 is selected from the group consisting of 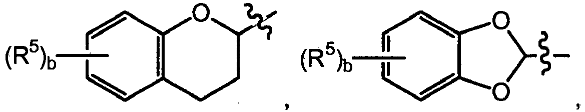 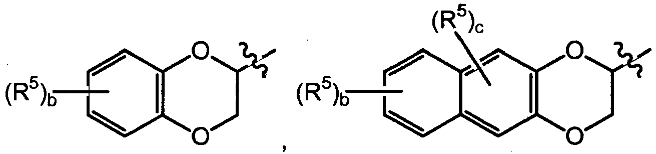 and 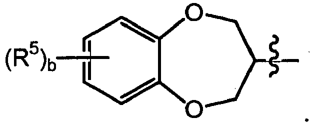 In another embodiment of the present invention, 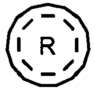 is selected from the group consisting of

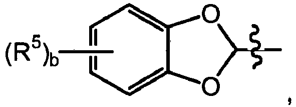

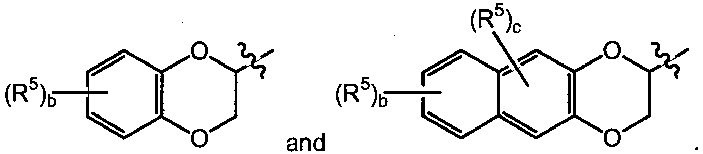

In an embodiment of the present invention, 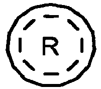 is selected from the group consisting of 2-(2,3-dihydro-benzo[1,4]dioxinyl), 2-(benzo[1,3]dioxolyl), 3-(3,4-dihydro-benzo[1,4]dioxepinyl), 2-(6-chloro-2,3-dihydro-benzo[1,4]dioxinyl), 2-(6-fluoro-2,3-dihydro-benzo[1,4]dioxinyl), 2-(chromanyl), 2-(5-fluoro-2,3-dihydro-benzo[1,4]dioxinyl), 2-(7-chloro-2,3-dihydro-benzo[1,4]dioxinyl), 2-(6-chloro-benzo[1,3]dioxolyl), 2-(7-nitro-2,3-dihydro-benzo[1,4]dioxinyl), 2-(7-methyl-2,3-dihydro-benzo[1,4]dioxinyl), 2-(5-chloro-2,3-dihydro-benzo[1,4]dioxinyl), 2-(6-bromo-2,3-dihydro-benzo[1,4]dioxinyl), 2-(6,7-dichloro-2,3-dihydro-benzo[1,4]dioxinyl), 2-(8-chloro-2,3-dihydro-benzo[1,4]dioxinyl), 2-(2,3-dihydro-naphtho[2,3-b][1,4]dioxinyl) and 2-(4-methyl-benzo[1,3]dioxolyl).

In another embodiment of the present invention, 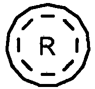 is selected from the group consisting 2-(benzo[1,3]dioxolyl), 2-(2,3-dihydro-benzo[1,4]dioxinyl), 2-(6-chloro-2,3-dihydro-benzo[1,4]dioxinyl), 2-(7-chloro-2,3-dihydro-benzo[1,4]dioxinyl), 2-(7-methyl-2,3-dihydro-benzo[1,4]dioxinyl), 2-(6-bromo-2,3-dihydro-benzo[1,4]dioxinyl) and 2-(6,7-dichloro-2,3-dihydro-benzo[1,4]dioxinyl). In another embodiment of the present invention, 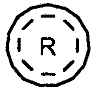 is selected from the group consisting of 2-(2,3-dihydro-benzo[1,4]dioxinyl), 2-(7-methyl-2,3-dihydro-benzo[1,4]dioxinyl) and 2-(6-bromo-2,3-dihydro-benzo[1,4]dioxinyl).

In an embodiment of the present invention R⁵ is selected from the group consisting of halogen and lower alkyl. In another embodiment of the present invention R⁵ is selected from chloro, fluoro, bromo and methyl.

In an embodiment of the present invention, the stereo-center on the compound of formula (I) is in the S-configuration. In another embodiment of the present invention, the stereo-center on the compound of formula (I) is in the R-configuration.

In an embodiment of the present invention the compound of formula (I) is present as an enantiomerically enriched mixture, wherein the % enantiomeric enrichment (% ee) is greater than about 75%, preferably greater than about 90%, more preferably greater than about 95%, most preferably greater than about 98%.

Additional embodiments of the present invention, include those wherein the substituents selected for one or more of the variables defined herein (i.e. R¹, R², R³, R⁴, X-Y and A) are independently selected to be any individual substituent or any subset of substituents selected from the complete list as defined herein.

Representative compounds of the present invention, are as listed in Tables 1 below. Additional compounds of the present invention are as listed in Table 3. In Tables 1 and 2 below, the column headed "stereo" defines the stereo-configuration at the carbon atom of the heterocycle attached at the starred bond. Where no designation is listed, the compound was prepared as a mixture of stereo-configurations. Where an "R" or "S" designation is listed, the stereo-configuration was based on the enantiomerically enriched starting material.

**Table 1: Representative Compounds of Formula (I)**

| | | | | | | |
|---|---|---|---|---|---|---|
| 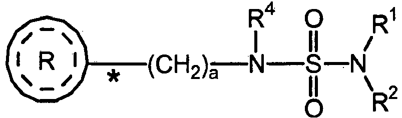 | | | | | | |

| **ID No.** | 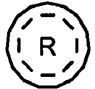 | **Stereo** | **(CH₂)ₐ** | **NR⁴** | **R¹** | **R²** |
|---|---|---|---|---|---|---|
| 1 | 2-(2,3-dihydro-benzo[1,4]dioxinyl) | | CH₂ | NH | H | H |
| 2 | 2-(benzo[1,3]dioxolyl) | | CH₂ | NH | H | H |
| 3 | 3-(3,4-dihydro-2H-benzo[1,4]dioxepinyl) | | CH₂ | NH | H | H |
| 4 | 2-(2,3-dihydro-benzo[1,4]dioxinyl) | S | CH₂ | NH | H | H |
| 5 | 2-(2,3-dihydro-benzo[1,4]dioxinyl) | R | CH₂ | NH | H | H |
| 6 | 2-(2,3-dihydro-benzo[1,4]dioxinyl) | | CH₂ | NH | methyl | methyl |
| 7 | 2-(2,3-dihydro-benzo[1,4]dioxinyl) | | CH₂ | N(CH₃) | H | H |
| 8 | 2-(6-chloro-2,3-dihydro-benzo[1,4]dioxinyl) | S | CH₂ | NH | H | H |
| 9 | 2-(6-fluoro-2,3-dihydro-benzo[1,4]dioxinyl) | S | CH₂ | NH | H | H |
| 10 | 2-(chromanyl) | | CH₂ | NH | H | H |
| 13 | 2-(5-fluoro-2,3-dihydro-benzo[1,4]dioxinyl) | S | CH₂ | NH | H | H |
| 14 | 2-(7-chloro-2,3-dihydro-benzo[1,4]dioxinyl) | S | CH₂ | NH | H | H |
| 15 | 2-(6-chloro-benzo[1,3]dioxolyl) | | CH₂ | NH | H | H |
| 16 | 2-(2,3-dihydro-benzo[1,4]dioxinyl) | | CH₂CH₂ | NH | H | H |
| 18 | 2-(7-nitro-2,3-dihydro-benzo[1,4]dioxinyl) | S | CH₂ | NH | H | H |
| 19 | 2-(7-methyl-2,3-dihydro-benzo[1,4]dioxinyl) | S | CH₂ | NH | H | H |
| 20 | 2-(5-chloro-2,3-dihydro-benzo[1,4]dioxinyl) | S | CH₂ | NH | H | H |
| 22 | 2-(8-methoxy-2,3-dihydro-benzo[1,4]dioxinyl) | S | CH₂ | NH | H | H |
| 24 | 2-(6-bromo-2,3-dihydro-benzo[1,4]dioxinyl) | S | CH₂ | NH | H | H |
| 29 | 2-(6,7-dichloro-2,3-dihydro-benzo[1,4]dioxinyl) | S | CH₂ | NH | H | H |
| 30 | 2-(8-chloro-2,3-dihydro-benzo[1,4]dioxinyl) | S | CH₂ | NH | H | H |
| 33 | 2-(2,3-dihydro-naphtho[2,3-b][1,4]dioxinyl) | S | CH₂ | NH | H | H |
| 35 | 2-(4-methyl-benzo[1,3]dioxolyl) | | CH₂ | NH | H | H |

**Table 2: Additional Compounds of the Present Invention**

| | | | | | | |
|---|---|---|---|---|---|---|
| 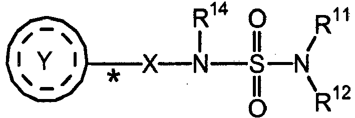 | | | | | | |

| **ID No.** | 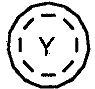 | **Stereo** | **X** | **NR¹⁴** | **R¹¹** | **R¹²** |
|---|---|---|---|---|---|---|
| 23 | 2-(5-methoxy-2,3-dihydro-benzo[1,4]dioxinyl) | S | CH₂ | NH | H | H |
| 26 | 2-(6-methylcarbonyl-2,3-dihydro-benzo[1,4]dioxinyl) | S | CH₂ | NH | H | H |
| 32 | 2-(6-methoxycarbonyl-2,3-dihydro-benzo[1,4]dioxinyl) | S | CH₂ | NH | H | H |
| 34 | 2-(6-hydroxymethyl-2,3-dihydro-benzo[1,4]dioxinyl) | S | CH₂ | NH | H | H |
| 36 | 2-(7-amino-2,3-dihydro-benzo[1,4]dioxinyl) | S | CH₂ | NH | H | H |

As used herein, unless otherwise noted, **"halogen"** shall mean chlorine, bromine, fluorine and iodine.

As used herein, unless otherwise noted, the term **"alkyl"** whether used alone or as part of a substituent group, includes straight and branched chains. For example, alkyl radicals include methyl, ethyl, propyl, isopropyl, butyl, isobutyl, sec-butyl, t-butyl, pentyl and the like. Unless otherwise noted, **"lower"** when used with alkyl means a carbon chain composition of 1-4 carbon atoms.

As used herein, unless otherwise noted, **"alkoxy"** shall denote an oxygen ether radical of the above described straight or branched chain alkyl groups. For example, methoxy, ethoxy, n-propoxy, sec-butoxy, t-butoxy, n-hexyloxy and the like.

As used herein, the notation "*" shall denote the presence of a stereogenic center.

When a particular group is **"substituted"** (e.g., alkyl, aryl, etc.), that group may have one or more substituents, preferably from one to five substituents, more preferably from one to three substituents, most preferably from one to two substituents, independently selected from the list of substituents.

With reference to substituents, the term **"independently"** means that when more than one of such substituents is possible, such substituents may be the same or different from each other.

Under standard nomenclature used throughout this disclosure, the terminal portion of the designated side chain is described first, followed by the adjacent functionality toward the point of attachment. Thus, for example, a **"phenyl-alkylamino-carbonyl-alkyl"** substituent refers to a group of the formula

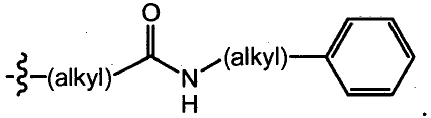

Abbreviations used in the specification, particularly the Schemes and Examples, are as follows:
- DCC: = Dicyclohexyl Carbodiimide
- DCE: = Dichloroethane
- DCM: = Dichloromethane
- DIPEA or DIEA: = Diisopropylethylamine
- DMF: = N,N-Dimethylformamide
- DMSO: = Dimethylsulfoxide
- EDC: = Ethylcarbodiimide
- Et₃N or TEA: = Triethylamine
- Et₂O: = Diethyl ether
- EA or EtOAc: = Ethyl acetate
- EtOH: = Ethanol
- IPA: = 2-propanol
- Hept: = Heptane
- HOBT: = 1-Hydroxybenzotriazole
- HPLC: = High Pressure Liquid Chromatography
- LAH: = Lithium Aluminum Hydride
- M or MeOH: = Methanol
- NMR: = Nuclear Magnetic Resonance
- Pd-C: = Palladium on Carbon Catalyst
- RP HPLC: = Reverse Phase High Pressure Liquid Chromatography
- RT or rt: = Room temperature
- TEA: = Triethylamine
- TFA: = Trifluoroacetic Acid
- THF: = Tetrahydrofuran
- TLC: = Thin Layer Chromatography

Where the compounds according to this invention have at least one chiral center, they may accordingly exist as enantiomers. Where the compounds possess two or more chiral centers, they may additionally exist as diastereomers. It is to be understood that all such isomers and mixtures thereof are encompassed within the scope of the present invention. Furthermore, some of the crystalline forms for the compounds may exist as polymorphs and as such are intended to be included in the present invention. In addition, some of the compounds may form solvates with water (i.e., hydrates) or common organic solvents, and such solvates are also intended to be encompassed within the scope of this invention.

For use in medicine, the salts of the compounds of this invention refer to non-toxic **"pharmaceutically acceptable salts."** Other salts may, however, be useful in the preparation of compounds according to this invention or of their pharmaceutically acceptable salts. Suitable pharmaceutically acceptable salts of the compounds include acid addition salts which may, for example, be formed by mixing a solution of the compound with a solution of a pharmaceutically acceptable acid such as hydrochloric acid, sulfuric acid, fumaric acid, maleic acid, succinic acid, acetic acid, benzoic acid, citric acid, tartaric acid, carbonic acid or phosphoric acid. Furthermore, where the compounds of the invention carry an acidic moiety, suitable pharmaceutically acceptable salts thereof may include alkali metal salts, e.g., sodium or potassium salts; alkaline earth metal salts, e.g., calcium or magnesium salts; and salts formed with suitable organic ligands, e.g., quaternary ammonium salts. Thus, representative pharmaceutically acceptable salts include the following:
acetate, benzenesulfonate, benzoate, bicarbonate, bisulfate, bitartrate,
   borate, bromide, calcium edetate, camsylate, carbonate, chloride, clavulanate, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isothionate, lactate, lactobionate, laurate, malate, maleate, mandelate, mesylate, methylbromide, methylnitrate, methylsulfate, mucate, napsylate, nitrate, N-methylglucamine ammonium salt, oleate, pamoate (embonate), palmitate, pantothenate, phosphate/diphosphate, polygalacturonate, salicylate, stearate, sulfate, subacetate, succinate, tannate, tartrate, teoclate, tosylate, triethiodide and valerate.

Representative acids and bases which may be used in the preparation of pharmaceutically acceptable salts include the following:
acids including acetic acid, 2,2-dichloroactic acid, acylated amino acids, adipic acid, alginic acid, ascorbic acid, L-aspartic acid, benzenesulfonic acid, benzoic acid, 4-acetamidobenzoic acid, (+)-camphoric acid, camphorsulfonic acid, (+)-(1S)-camphor-10-sulfonic acid, capric acid, caproic acid, caprylic acid, cinnamic acid, citric acid, cyclamic acid, dodecylsulfuric acid, ethane-1,2-disulfonic acid, ethanesulfonic acid, 2-hydrocy-ethanesulfonic acid, formic acid, fumaric acid, galactaric acid, gentisic acid, glucoheptonic acid, D-gluconic acid, D-glucoronic acid, L-glutamic acid, α-oxo-glutaric acid, glycolic acid, hipuric acid, hydrobromic acid, hydrochloric acid, (+)-L-lactic acid, (±)-DL-lactic acid, lactobionic acid, maleic acid, (-)-L-malic acid, malonic acid, (±)-DL-mandelic acid, methanesulfonic acid, naphthalene-2-sulfonic acid, naphthalene-1,5-disulfonic acid, 1-hydroxy-2-naphthoic acid, nicotinc acid, nitric acid, oleic acid, orotic acid, oxalic acid, palmitric acid, pamoic acid, phosphoric acid, L-pyroglutamic acid, salicylic acid, 4-amino-salicylic acid, sebaic acid, stearic acid, succinic acid, sulfuric acid, tannic acid, (+)-L-tartaric acid, thiocyanic acid, p-toluenesulfonic acid and undecylenic acid; and
bases including ammonia, L-arginine, benethamine, benzathine, calcium hydroxide, choline, deanol, diethanolamine, diethylamine, 2-(diethylamino)-ethanol, ethanolamine, ethylenediamine, N-methyl-glucamine, hydrabamine, 1H-imidazole, L-lysine, magnesium hydroxide, 4-(2-hydroxyethyl)-morpholine, piperazine, potassium hydroxide, 1-(2-hydroxyethyl)-pyrrolidine, secondary amine, sodium hydroxide, triethanolamine, tromethamine and zinc hydroxide.

Compounds of formula (I) may be prepared according to the process outlined in Scheme 1.

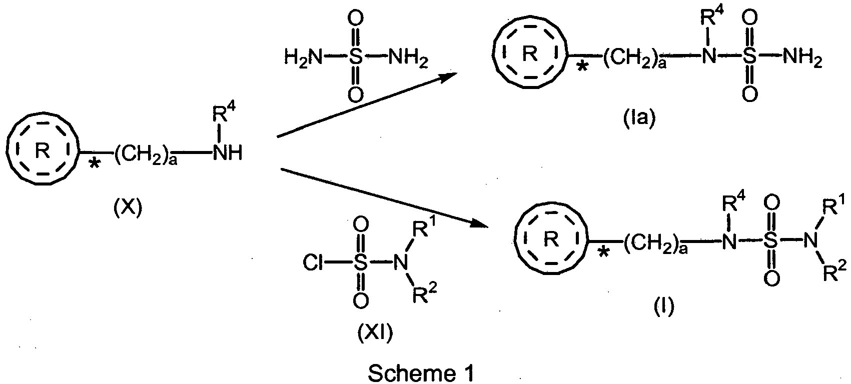

Accordingly, a suitably substituted compound of formula (X), a known compound or compound prepared by known methods, is reacted with sulfamide, a known compound, preferably wherein the sulfamide is present in an amount in the range of about 2 to about 5 equivalents, in an organic solvent such as THF, dioxane, and the like, preferably at an elevated temperature in the range of about 50°C to about 100°C, more preferably at about reflux temperature, to yield the corresponding compound of formula (Ia).

Alternatively, a suitably substituted compound of formula (X), a known compound or compound prepared by known methods, is reacted with a suitably substituted compound of formula (XI), a known compound or compound prepared by known methods, in the presence of a base such as TEA, DIPEA, pyridine, and the like, in an organic solvent such as DMF, DMSO, and the like, to yield the corresponding compound of formula (I).

Compounds of formula (X) wherein 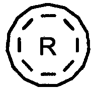 is 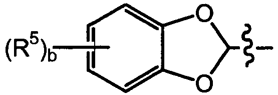 may be prepared according to the process outlined in Scheme 2.

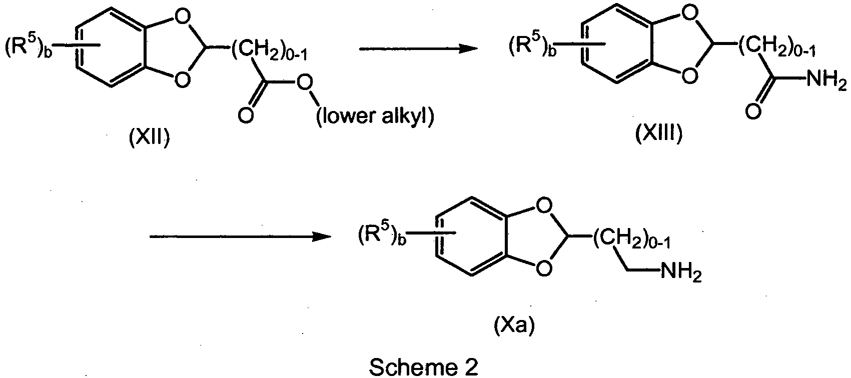

Accordingly, a suitably substituted compound of formula (XII), a known compound or compound prepared by known method (for example as described in Scheme 3 above) is reacted with NH₄OH, a known compound, optionally in an organic solvent such as acetonitrile, and the like, to yield the corresponding compound of formula (XIII).

The compound of formula (XIII) is reacted with a suitably selected reducing agent, such as LAH, and the like, and the like, in an organic solvent such as THF, diethyl ether, and the like, to yield the corresponding compound of formula (Xa).

Compounds of formula (X) wherein 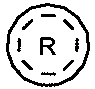 is selected from 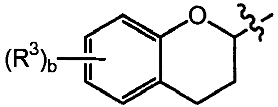 may be prepared according to the process outlined in Scheme 3.

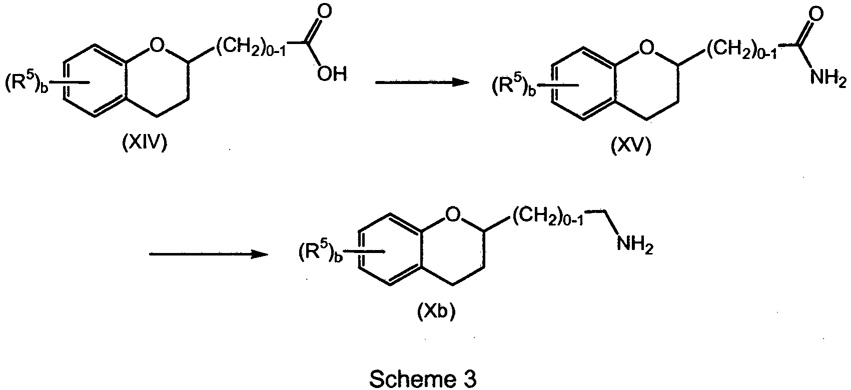

Accordingly, a suitably substituted compound of formula (XIV), a known compound or compound prepared by known methods, is reacted with NH₄OH, in the presence of a coupling agent such as DCC, and the like, optionally in an organic solvent such as acetonitrile, and the like, to yield the corresponding compound of formula (XV).

The compound of formula (XV) is reacted with a suitably selected reducing agent, such as LAH, and the like, in an organic solvent such as THF, diethyl ether, and the like, to yield the corresponding compound of formula (Xb).

Compounds of formula (X) wherein 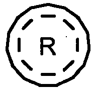 is selected from 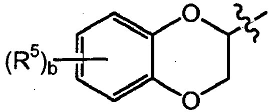 and wherein a is 2, may be prepared according to the process outlined in Scheme 4.

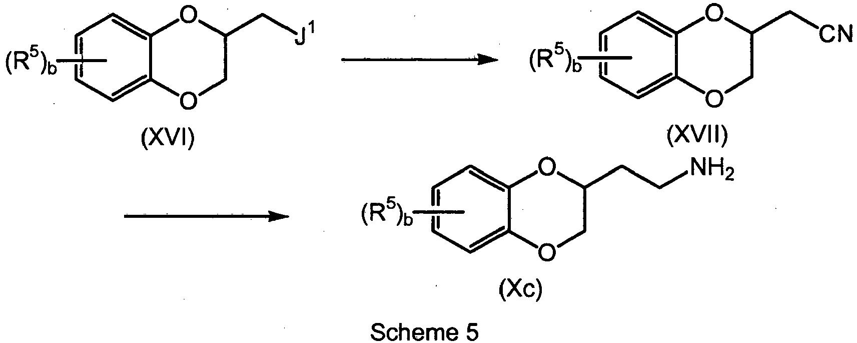

Accordingly, a suitably substituted compound of formula (XVI) wherein J¹ is a suitable leaving group such as Br, Cl, I, tosyl, mesyl, triflyl, and the like, a known compound or compound prepared by known methods (for example, by activating the corresponding compound wherein J¹ is OH), is reacted with a cyanide such as potassium cyanide, sodium cyanide, and the like, in an organic solvent such as DMSO, DMF, THF, and the like, to yield the corresponding compound of formula (XVII).

The compound of formula (XVII) is reduced according to known methods, for example by reacting with a suitable reducing agent such as LAH, borane, and the like, to yield the corresponding compound of formula (Xc).

Compounds of formula (X) wherein 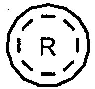 is selected from 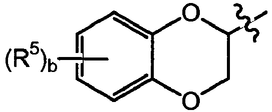 and wherein a is 1, may be prepared according to the process outlined in Scheme 5.

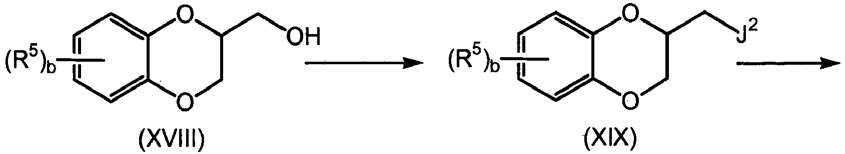

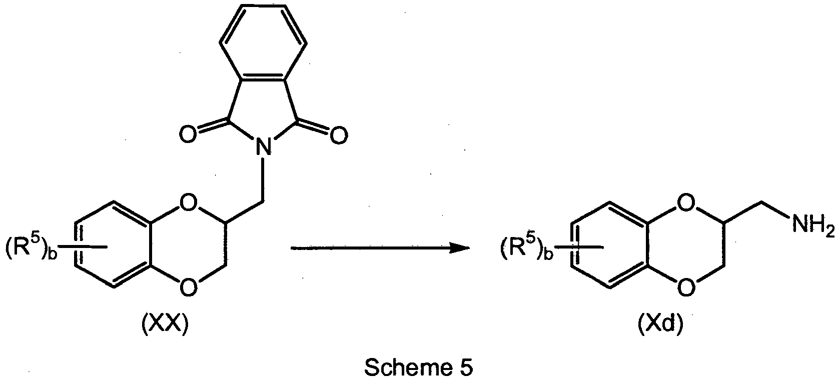

Accordingly, a suitably substituted compound of formula (XVIII), a known compound or compound prepared by known methods is activated, according to known method, to yield the corresponding compound of formula (XIX), wherein J² is a suitable leaving group, such tosylate, Cl, Br, I, mesylate, triflate, and the like.

The compound of formula (XIX) is reacted with a phthalimide salt such as potassium phthlimide, sodium phthalimide, and the like, in an organic solvent such as DMF, DMSO, acetonitrile, and the like, preferably, at an elevated temperature in the range of from 50°C to about 200°C, more preferably, at about reflux temperature, to yield the corresponding compound of formula (XX).

The compound of formula (XX) is reacted with N₂H₄, a known compound, in an organic solvent such as ethanol, methanol, and the like, preferably, at an elevated temperature in the range of from about 50°C to about 100°C, more preferably, at about reflux temperature, and the like, to yield the corresponding compound of formula (Xd).

One skilled in the art will recognize that compounds of formula (X) wherein 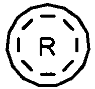 is selected from 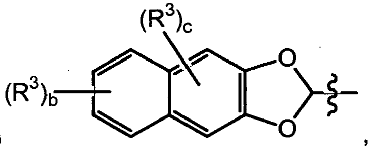 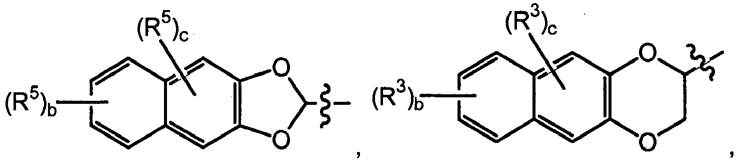 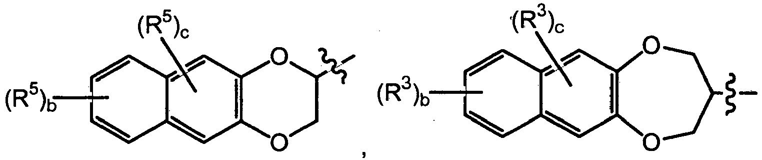 or 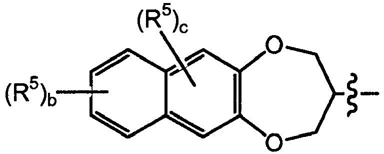 may be similarly prepared according to known methods or for example, according to the processes outlined in Schemes 2 through 5 above, by selecting and substituting the corresponding naphthyl-fused compounds for the benzo-fused starting materials.

One skilled in the art will further recognize that wherein a single enantiomer (or a mixture of enantiomers wherein one enantiomer is enriched) of a compound of formula (X) is desired, the above processes as described in Schemes 1 through 5 may be applied by substituting the corresponding single enantiomer (or mixture of enantiomers wherein one enantiomer is enriched) for the appropriate starting material.

One skilled in the art will recognize that wherein a reaction step of the present invention may be carried out in a variety of solvents or solvent systems, said reaction step may also be carried out in a mixture of the suitable solvents or solvent systems.

Where the processes for the preparation of the compounds according to the invention give rise to mixture of stereoisomers, these isomers may be separated by conventional techniques such as preparative chromatography. The compounds may be prepared in racemic form, or individual enantiomers may be prepared either by enantiospecific synthesis or by resolution. The compounds may, for example, be resolved into their component enantiomers by standard techniques, such as the formation of diastereomeric pairs by salt formation with an optically active acid, such as (-)-di-p-toluoyl-D-tartaric acid and/or (+)-di-p-toluoyl-L-tartaric acid followed by fractional crystallization and regeneration of the free base. The compounds may also be resolved by formation of diastereomeric esters or amides, followed by chromatographic separation and removal of the chiral auxiliary. Alternatively, the compounds may be resolved using a chiral HPLC column.

During any of the processes for preparation of the compounds of the present invention, it may be necessary and/or desirable to protect sensitive or reactive groups on any of the molecules concerned. This may be achieved by means of conventional protecting groups, such as those described in Protective Groups in Organic Chemistry, ed. J.F.W. McOmie, Plenum Press, 1973; and T.W. Greene & P.G.M. Wuts, Protective Groups in Organic Synthesis, John Wiley & Sons, 1991. The protecting groups may be removed at a convenient subsequent stage using methods known from the art.

The present invention further comprises pharmaceutical compositions containing one or more compounds of formula (I) with a pharmaceutically acceptable carrier. Pharmaceutical compositions containing one or more of the compounds of the invention described herein as the active ingredient can be prepared by intimately mixing the compound or compounds with a pharmaceutical carrier according to conventional pharmaceutical compounding techniques. The carrier may take a wide variety of forms depending upon the desired route of administration (e.g., oral, parenteral). Thus for liquid oral preparations such as suspensions, elixirs and solutions, suitable carriers and additives include water, glycols, oils, alcohols, flavoring agents, preservatives, stabilizers, coloring agents and the like; for solid oral preparations, such as powders, capsules and tablets, suitable carriers and additives include starches, sugars, diluents, granulating agents, lubricants, binders, disintegrating agents and the like. Solid oral preparations may also be coated with substances such as sugars or be enteric-coated so as to modulate major site of absorption. For parenteral administration, the carrier will usually consist of sterile water and other ingredients may be added to increase solubility or preservation. Injectable suspensions or solutions may also be prepared utilizing aqueous carriers along with appropriate additives.

To prepare the pharmaceutical compositions of this invention, one or more compounds of the present invention as the active ingredient is intimately admixed with a pharmaceutical carrier according to conventional pharmaceutical compounding techniques, which carrier may take a wide variety of forms depending of the form of preparation desired for administration, e.g., oral or parenteral such as intramuscular. In preparing the compositions in oral dosage form, any of the usual pharmaceutical media may be employed. Thus, for liquid oral preparations, such as for example, suspensions, elixirs and solutions, suitable carriers and additives include water, glycols, oils, alcohols, flavoring agents, preservatives, coloring agents and the like; for solid oral preparations such as, for example, powders, capsules, caplets, gelcaps and tablets, suitable carriers and additives include starches, sugars, diluents, granulating agents, lubricants, binders, disintegrating agents and the like. Because of their ease in administration, tablets and capsules represent the most advantageous oral dosage unit form, in which case solid pharmaceutical carriers are obviously employed. If desired, tablets may be sugar coated or enteric coated by standard techniques. For parenterals, the carrier will usually comprise sterile water, through other ingredients, for example, for purposes such as aiding solubility or for preservation, may be included. Injectable suspensions may also be prepared, in which case appropriate liquid carriers, suspending agents and the like may be employed. The pharmaceutical compositions herein will contain, per dosage unit, e.g., tablet, capsule, powder, injection, teaspoonful and the like, an amount of the active ingredient necessary to deliver an effective dose as described above. The pharmaceutical compositions herein will contain, per unit dosage unit, e.g., tablet, capsule, powder, injection, suppository, teaspoonful and the like, of from about 0.1-1000 mg and may be given at a dosage of from about 0.01-200.0 mg/kg/day, preferably from about 0.1 to 100 mg/kg/day, more preferably from about 0.5-50 mg/kg/day, more preferably from about 1.0-25.0 mg/kg/day or any range therein. The dosages, however, may be varied depending upon the requirement of the patients, the severity of the condition being treated and the compound being employed. The use of either daily administration or post-periodic dosing may be employed.

Preferably these compositions are in unit dosage forms from such as tablets, pills, capsules, powders, granules, sterile parenteral solutions or suspensions, metered aerosol or liquid sprays, drops, ampoules, autoinjector devices or suppositories; for oral parenteral, intranasal, sublingual or rectal administration, or for administration by inhalation or insufflation. Alternatively, the composition may be presented in a form suitable for once-weekly or once-monthly administration; for example, an insoluble salt of the active compound, such as the decanoate salt, may be adapted to provide a depot preparation for intramuscular injection. For preparing solid compositions such as tablets, the principal active ingredient is mixed with a pharmaceutical carrier, e.g. conventional tableting ingredients such as corn starch, lactose, sucrose, sorbitol, talc, stearic acid, magnesium stearate, dicalcium phosphate or gums, and other pharmaceutical diluents, e.g. water, to form a solid preformulation composition containing a homogeneous mixture of a compound of the present invention, or a pharmaceutically acceptable salt thereof. When referring to these preformulation compositions as homogeneous, it is meant that the active ingredient is dispersed evenly throughout the composition so that the composition may be readily subdivided into equally effective dosage forms such as tablets, pills and capsules. This solid preformulation composition is then subdivided into unit dosage forms of the type described above containing from 0.1 to about 1000 mg of the active ingredient of the present invention. The tablets or pills of the novel composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of material can be used for such enteric layers or coatings, such materials including a number of polymeric acids with such materials as shellac, cetyl alcohol and cellulose acetate.

The liquid forms in which the novel compositions of the present invention may be incorporated for administration orally or by injection include, aqueous solutions, suitably flavored syrups, aqueous or oil suspensions, and flavored emulsions with edible oils such as cottonseed oil, sesame oil, coconut oil or peanut oil, as well as elixirs and similar pharmaceutical vehicles. Suitable dispersing or suspending agents for aqueous suspensions, include synthetic and natural gums such as tragacanth, acacia, alginate, dextran, sodium carboxymethylcellulose, methylcellulose, polyvinyl-pyrrolidone or gelatin.

The method of treating anxiety and related disorders described in the present invention may also be carried out using a pharmaceutical composition comprising any of the compounds as defined herein and a pharmaceutically acceptable carrier. The pharmaceutical composition may contain between about 0.1 mg and 1000 mg, preferably about 50 to 500 mg, of the compound, and may be constituted into any form suitable for the mode of administration selected. Carriers include necessary and inert pharmaceutical excipients, including, but not limited to, binders, suspending agents, lubricants, flavorants, sweeteners, preservatives, dyes, and coatings. Compositions suitable for oral administration include solid forms, such as pills, tablets, caplets, capsules (each including immediate release, timed release and sustained release formulations), granules, and powders, and liquid forms, such as solutions, syrups, elixers, emulsions, and suspensions. Forms useful for parenteral administration include sterile solutions, emulsions and suspensions.

Advantageously, compounds of the present invention may be administered in a single daily dose, or the total daily dosage may be administered in divided doses of two, three or four times daily. Furthermore, compounds for the present invention can be administered in intranasal form via topical use of suitable intranasal vehicles, or via transdermal skin patches well known to those of ordinary skill in that art. To be administered in the form of a transdermal delivery system, the dosage administration will, of course, be continuous rather than intermittent throughout the dosage regimen.

For instance, for oral administration in the form of a tablet or capsule, the active drug component can be combined with an oral, non-toxic pharmaceutically acceptable inert carrier such as ethanol, glycerol, water and the like. Moreover, when desired or necessary, suitable binders; lubricants, disintegrating agents and coloring agents can also be incorporated into the mixture. Suitable binders include, without limitation, starch, gelatin, natural sugars such as glucose or beta-lactose, corn sweeteners, natural and synthetic gums such as acacia, tragacanth or sodium oleate, sodium stearate, magnesium stearate, sodium benzoate, sodium acetate, sodium chloride and the like. Disintegrators include, without limitation, starch, methyl cellulose, agar, bentonite, xanthan gum and the like.

The liquid forms in suitably flavored suspending or dispersing agents such as the synthetic and natural gums, for example, tragacanth, acacia, methylcellulose and the like. For parenteral administration, sterile suspensions and solutions are desired. Isotonic preparations which generally contain suitable preservatives are employed when intravenous administration is desired.

Compounds of this invention may be administered in any of the foregoing compositions and according to dosage regimens established in the art whenever treatment of anxiety and related disorders is required.

The daily dosage of the products may be varied over a wide range from 0.01 to 200 mg / kg per adult human per day. For oral administration, the compositions are preferably provided in the form of tablets containing, 0.01, 0.05, 0.1, 0.5, 1.0, 2.5, 5.0, 10.0, 15.0, 25.0, 50.0, 100, 150, 200, 250, 500 and 1000 milligrams of the active ingredient for the symptomatic adjustment of the dosage to the patient to be treated. An effective amount of the drug is ordinarily supplied at a dosage level of from about 0.01 mg/kg to about 200 mg/kg of body weight per day, or any range therein. Preferably, the range is from about 0.1 to about 100.0 mg/kg of body weight per day, more preferably, from about 0.5 mg/kg to about 50 mg/kg, more preferably, from about 1.0 to about 25.0 mg/kg of body weight per day. The compounds may be administered on a regimen of 1 to 4 times per day.

Optimal dosages to be administered may be readily determined by those skilled in the art, and will vary with the particular compound used, the mode of administration, the strength of the preparation, the mode of administration, and the advancement of the disease condition. In addition, factors associated with the particular patient being treated, including patient age, weight, diet and time of administration, will result in the need to adjust dosages.

One skilled in the art will recognize that, both *in vivo* and *in vitro* trials using suitable, known and generally accepted cell and / or animal models are predictive of the ability of a test compound to treat or prevent a given disorder.

One skilled in the art will further recognize that human clinical trails including first-in-human, dose ranging and efficacy trials, in healthy patients and / or those suffering from a given disorder, may be completed according to methods well known in the clinical and medical arts.

The following Examples are set forth to aid in the understanding of the invention, and are not intended and should not be construed to limit in any way the invention set forth in the claims which follow thereafter.

### Example 1

### ((3,4-Dihydro-2H-benzo[b][1,4]dioxepin-3-yl)methyl)sulfamide (Compound #3)

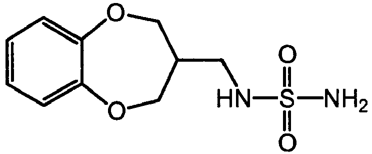

Catechol (5.09 g, 46.2 mmol) and potassium carbonate were combined in acetonitrile and heated to reflux for one hour. 2-Chloromethyl-3-chloro-1-propene (5.78 g, 46.2 mmol) was added and the reaction was continued at reflux for 24 hours. The solution was cooled to room temperature and filtered. The filtrate was evaporated and the residue was diluted with water and extracted with diethyl ether (3 x). The combined organic solution was dried over MgSO₄ and concentrated. Chromatography (2% ethyl ether in hexane) yielded 3-methylene-3,4-dihydro-2H-benzo[b][1,4]dioxepine as a colorless oil.
MS (ESI): 163.2 (M+H⁺)
¹H NMR (300 MHz, CDCl₃), δ: 6.94 (m, 4H), 5.07 (s, 2H), 4.76 (s, 4H).

3-Methylene-3,4-dihydro-2H-benzo[b][1,4]dioxepine (5.00 g, 30.8 mmol) was dissolved in dry THF (100 mL). Borane-THF (1.0 M in THF, 10.3 mL) was added at 0°C. The reaction was stirred at RT for 5 hours. Aminosulfonic acid (6.97 g, 61.6 mmol) was added. The reaction was heated to reflux overnight. The reaction was cooled to room temperature and aqueous sodium hydroxide (3.0 M, 100 mL) was added. The solution was extracted with ethyl acetate (3 x 100 mL). The combined organic solution was dried over MgSO₄. The solution was concentrated under vacuum and purified by chromatography (2% to 8% methanol in dichloromethane) to yield ((3,4-dihydro-2H-benzo[b][1,4]dioxepin-3-yl)methyl)amine as a colorless oil.
MS (ESI): 180.1 (M+H⁺)
¹H NMR (300 MHz, DMSO), δ: 6.92 (m, 4H), 4.21 (m, 2H), 4.07 (m, 2H), 3.33 (broad, 2H), 3.16 (d, J = 4 Hz, 1H), 2.72 (d, J = 4 Hz, 1 H), 2.30 (m, 1 H).

((3,4-Dihydro-2H-benzo[b][1,4]dioxepin-3-yl)methyl)amine (2.90 g, 16.2 mmol) and sulfamide (3.11 g, 32.4 mmol) were combined in dry dioxane (60 ml) and heated to reflux overnight. Chloroform was added and the precipitate was removed by filtration. The filtrate was concentrated under vacuum and purified by chromatography (2% to 8% acetone in dichloromethane) to yield the title compound as an off-white solid.
258.8 (M+H⁺)
¹H NMR (300 MHz, DMSO), δ: 6.92 (m, 4H), 6.71 (broad, 1 H), 6.59 (broad, 2H), 4.19 (m, 2H), 4.04 (m, 2H), 3.00 (m, 2H), 2.39 (m, 1 H).

### Example 2

### N-(2,3-Dihydro-benzo[1,4]dioxin-2-ylmethyl)-sulfamide (Compound #1)

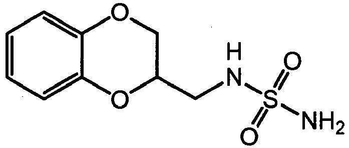

Racemic 2,3-dihydro-1,4-benzdioxin-2-ylmethylamine (4.4 g, 26 mmol) and sulfamide (5.1 g, 53 mmol) were combined in 1,4 dioxane (100 mL) and refluxed for 2 h. The reaction was cooled to room temperature and a small amount of solid was filtered and discarded. The filtrate was evaporated in vacuo and the residue was purified using flash column chromatography (DCM:Methanol - 10:1) to yield a white solid. The solid was recrystallized from DCM to yield the title compound as a white solid.
mp: 97.5 - 98.5°C
Elemental Analysis:

| | |
|---|---|
| Anal Calc: | C, 44.25; H, 4.95; N, 11.47; S, 13.13 |
| Anal Found: | C, 44.28; H, 4.66; N, 11.21; S, 13.15 |

H¹ NMR (DMSO d6) δ 6.85 (m, 4H), 6.68 (bd s, 3H, NH), 4.28 (m, 2H), 3.97 (dd, J = 6.9, 11.4 Hz, 1 H), 3.20 (m, 1H), 3.10 (m, 1 H).

### Example 3

### (Benzo[1,3]dioxol-2-ylmethyl)sulfamide (Compound #2)

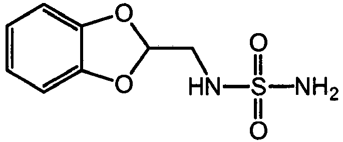

Catechol (10.26 g, 93.2 mmol), sodium methoxide (25% by weight in methanol, 40.3 g, 186 mmol), and methyl dichloroacetate (13.3 g, 93.2 mmol) were combined in dry methanol (100 mL). The solution was heated to reflux overnight. The reaction was cooled to room temperature, acidified by addition of concentrated hydrochloric acid and then reduced in volume under vacuum to about 50 mL. Water was added and the mixture was extracted with diethyl ether (3 x 100 mL). The combined organic solution was dried with MgSO₄, concentrated to a brown solid, and chromatographed (2% ethyl acetate in hexane) to yield benzo[1,3]dioxole-2-carboxylic acid methyl ester as a colorless oil.
MS (ESI): 195.10 (M+H⁺).
¹H NMR (300 MHz, CDCl₃), δ: 6.89 (broad, 4H), 6.29 (s, 1 H), 4.34 (q, J =7 Hz, 2H), 1.33 (t, J =7 Hz, 3H).

To benzo[1,3]dioxole-2-carboxylic acid methyl ester (7.21 g, 40.0 mmol) was added ammonium hydroxide (29% in water, 10 mL) and enough acetonitrile to make the mixture homogeneous (∼5 mL). The solution was stirred for two hours at room temperature and then distilled water was added. Benzo[1,3]dioxole-2-carboxylic acid amide precipitated as a white solid and was collected by filtration and used without further purification.
MS (ESI): 160.00 (M+H⁺)
¹H NMR (300 MHz, DMSO), δ: 7.99 (s, broad, 1 H), 7.72 (s, broad, 1 H), 6.94 (m, 2H) 6.86 (m, 2H), 6.30 (s, 1 H).

Benzo[1,3]dioxole-2-carboxylic acid amide (5.44 g, 32.9 mmol) was dissolved in tetrahydrofuran (THF, 100 mL). Lithium aluminum hydride (LAH, 1 M in THF, 39.5 mL, 39.5 mmol) was added slowly to the solution at room temperature. The reaction was stirred at room temperature for 24 hours. Distilled water was added to destroy the excess LAH. Aqueous sodium hydroxide (3.0 M, 100 mL) was added and the solution was extracted with ethyl acetate (3 x 100 mL). The combined organic solution was washed with water and dried over MgSO₄. The solvent was evaporated to yield C-benzo[1,3]dioxol-2-yl-methylamine as a colorless oil.
MS (ESI): 152.1 (M+H⁺)
¹H NMR (300 MHz, CDCl₃), δ: 6.87 (m, 4H), 6.09 (t, J = 4 Hz, 1 H), 3.13 (d, J = 4 Hz, 2H)

C-Benzo[1,3]dioxol-2-yl-methylamine (2.94 g, 19.4 mmol) and sulfamide (3.74 g, 38.9 mmol) were combined in dry dioxane (50 mL) and the solution was heated to reflux overnight. The reaction was concentrated and the residue was chromatographed (2% to 10% acetone in dichloromethane) to yield the title compound as a white solid.
MS (ESI): 230.0 (M+H⁺)
¹H NMR (300 MHz, CDCl₃), δ: 6.87 (m, 4H), 6.25 (t, J = 4 Hz, 1 H), 4.79 (broad, 1 H), 4.62 (broad, 1 H), 3.64 (d, J = 4 Hz, 2H).

### Example 4

### (2S)-(-)-N-(2,3-Dihydro-benzo[1,4]dioxin-2-ylmethyl)-sulfamide (Compound #4)

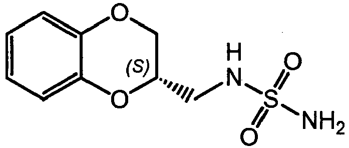

Catechol (13.2 g, 0.12 mol) and potassium carbonate (16.6 g, 0.12 mol) were stirred in DMF (250 mL) and (2R)-glycidyl tosylate (22.8 g, 0.10 mol) was added and the reaction was stirred at 60°C for 24 h. The reaction was cooled to room temperature and diluted with ice water (1 L) and extracted with diethyl ether (4 times). The combined organic solution was washed 3 times with 10% potassium carbonate, once with water, once with brine and evaporated in vacuo to yield a white solid which was purified by flash column chromatography (DCM:Methanol - 50:1) to yield ((2S)-2,3-dihydro-benzo[1,4]dioxin-2-yl)-methanol as a solid.

The solid (13.3 g, 68 mmol) was dissolved in pyridine (85 mL) cooled to 0°C, p-toluenesulfonyl chloride (13.0 g, 68 mmol) was added and the reaction mixture stirred at room temperature for 20h. The reaction was diluted with diethyl ether (1 L) and 1 N HCl (1.2 L). The organic layer was separated and washed 2 times with 1 N HCl (500 mL), 4 times with water (150 mL), once with brine, dried (MgSO₄) and evaporated in vacuo to yield a white solid which was purified by flash column chromatography (Hept:EA - 2:1) to yield toluene-4-sulfonic acid (2S)-2,3-dihydro-benzo[1,4]dioxin-2-ylmethyl ester as a white solid.

The white solid was combined with potassium phthalimide (14.4 g, 78 mmol) in DMF (250 mL) and heated to reflux for 1 h, cooled to room temperature and poured into vigorously stirring water (1.5 L) and stirred 30 min. White solid was filtered and the solid was washed several times with water, 2% NaOH, and water again and let air dry to yield a (2S)-2-(2,3-Dihydro-benzo[1,4]dioxin-2-ylmethyl)-isoindole-1,3-dione as white powdery solid.

The powdery white solid was combined with hydrazine (2.75 g, 86 mmol) in EtOH (225 mL) and heated at reflux for 2 h, cooled to room temperature and 1 N HCl added to pH 1.0 and stirred for 15 min. White solid was filtered and washed with fresh EtOH (solid discarded) and the filtrate was evaporated in vacuo to a solid, which was partitioned between diethyl ether and dilute aqueous NaOH. The diethyl ether solution was dried (Na₂SO₄) and evaporated in vacuo to a yield a light yellow oil. The oil was purified by flash column chromatography (DCM:MeOH - 10:1) to yield an oil. A portion of the oil (4.82 g, 29 mmol) in 2-propanol (250 mL) was treated with 1 N HCl (30 mL) and heated on steambath until homogeneous and then let cool to room temperature. After 3 h, the mixture was ice cooled for 2 h. A white flaky solid

(the corresponding HCl salt of (2S)-C-(2,3-Dihydro-benzo[1,4]dioxin-2-yl)-methylamine) was filtered off and then recrystallized again from 2-propanol to yield a white solid.
[a]_{D} = -69.6 (c = 1.06, EtOH)

The white solid was partitioned between DCM and dilute NaOH, and the DCM was dried (NaSO₄) and evaporated in vacuo to yield (2S)-C-(2,3-Dihydro-benzo[1,4]dioxin-2-yl)-methylamine as an oil.
[α]_{D} = -57.8 (c = 1.40, CHCl₃)

The oil (2.1 g, 12.7 mmol) and sulfamide (2.44 g, 25.4 mmol) were refluxed in dioxane (75 mL) for 2 h and the crude product was purified by flash column chromatography (DCM:MeOH 10:1) to yield a white solid, which was recrystallized from DCM to yield the title compound as a white crystalline solid.
mp 102-103°C
[α]_{D} = -45.1° (c = 1.05, M);
¹H NMR (DMSOd6) δ 6.86 (m, 4H), 6.81 (bd s, 3H, NH), 4.3 (m, 2H), 3.97 (dd, J = 6.9, 11.4 Hz, 1H), 3.20 (dd, J = 5.5, 13.7 Hz, 1 H), 3.10 (dd, J = 6.9, 13.7 Hz, 1H)
Elemental Analysis:

| | |
|---|---|
| Anal Calc: | C, 44.25; H, 4.95; N, 11.47; S, 13.13 |
| Anal Found: | C, 44.20; H, 4.69; N, 11.40; S, 13.22. |

### Example 5

### N-(2,3-Dihydro-benzo[1,4]dioxin-2-ylmethyl)-N',N'dimethylsulfamide (Compound #6)

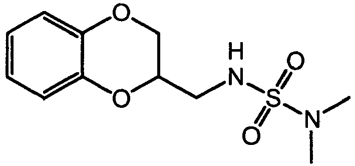

Racemic 2,3-dihydro-1,4-benzdioxin-2-ylmethylamine (8.25 g, 5.0 mmol) and triethylamine (1.52 g, 15 mmol) were combined in DMF (10 mL) and cooled in an ice bath as dimethylsulfamoyl chloride (1.44 g, 10 mmol) was added. The reaction mixture was then stirred for 3 hr with continued cooling. The reaction mixture was partitioned between ethyl acetate and water, and the ethyl acetate solution was washed with brine, dried (MgSO₄) and evaporated in vacuo to yield an oil. The oil was purified using flash column chromatography (ethyl acetate:Heptane - 1:1) to yield a white solid, which was recrystallized (ethyl acetate/Hexane) to yield the title compound as a white floccular solid.
mp 76 - 78°C
MS 273 (MH⁺)
Elemental Analysis:

| | |
|---|---|
| Anal Calc: | C, 48.52; H, 5.92; N, 10.29; S, 11.78 |
| Anal Found: | C, 48.63; H, 5.62; N, 10.20; S, 11.90 |

¹H NMR (CDCl₃) δ 6.87 (m, 4H), 4.59 (bd m, 1 H, NH), 4.35 (m, 1 H), 4.27 (dd, J = 2.3, 11.4 Hz, 1 H), 4.04 (dd, J = 7.0, 11.4, 1H), 3.36 (m, 2H), 2.82 (s, 6H).

### Example 6

### N-(2,3-Dihydro-benzo[1,4]dioxin-2-ylmethyl)-N-methylsulfamide (Compound #7)

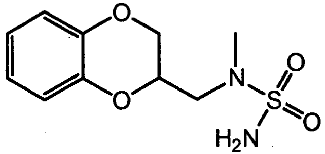

Racemic 2,3-dihydro-1,4-benzdioxin-2-ylmethylamine (825 mg, 5 mmol) was dissolved in ethyl formate (15 mL), refluxed for 30 min and evaporated in vacuo to yield N-(2,3-dihydro-benzo[1,4]dioxin-2-ylmethyl)-formamide as an oil.

The oil in diethyl ether (25 mL) was treated with 1 M LAH in THF (9.0 mL, 9.0 mmol) at 0°C and stirred for 5 h at room temperature. The reaction was cooled in an ice bath and quenched with water (0.50 mL), followed by 3 N NaOH (0.50 mL) and water (0.50 mL). The mixture was then stirred at room temperature for 1 h. Solid was filtered and the filtrate was evaporated in vacuo to yield a residue which was partitioned between 1 N HCl and diethyl ether. The aqueous phase was basified with 1 N NaOH and extracted with diethyl ether. The organic phase was dried (MgSO₄) and evaporated in vacuo to yield (2,3-dihydro-benzo[1,4]dioxin-2-ylmethyl)-methyl-amine as an oil.
MS 180 (MH⁺)
¹H NMR (CDCl₃) δ 6.85 (m, 4H), 4.30 (m, 2H), 4.02 (dd, J = 7.9, 11.6 Hz, 1 H), 2.85 (m, 2H), 2.50 (s, 3H)

The oil (380 mg, 2.1 mmol) and sulfamide (820 mg, 8.5 mmol) were combined in dioxane (15 mL), refluxed for 1.5 h and evaporated in vacuo to yield a crude residue. The residue was purified via column chromatography (ethyl acetate/Heptane 1:1) and the resultant solid was recrystallized from ethyl acetate/Hexane to yield the title compound as a white solid.
mp 97-98°C
MS 257 (M⁻¹)
Elemental Analysis:

| | |
|---|---|
| Anal Calc: | C, 46.50; H, 5.46; N, 10.85; S, 12.41 |
| Anal Found: | C, 46.48; H, 5.65; N, 10.90; S, 12.07 |

¹H NMR (CDCl₃) δ 6.86 (m, 4H), 4.52 (bs, 2H), 4.46 (m, 1 H), 4.29 (dd, J = 2.3, 11.5 Hz, 1 H), 4.05 (dd, J = 6.5, 11.5 Hz, 1 H), 3.51 (dd, J = 6.7, 14.9 Hz, 1 H), 3.40 (dd, J = 5.9, 14.9 Hz, 1H), 2.99 (s, 3H).

### Example 7

### (2S)-(-)-N-(6-Chloro-2,3-dihydro-benzo[1,4]dioxin-2-ylmethyl)-sulfamide (Compound #8)

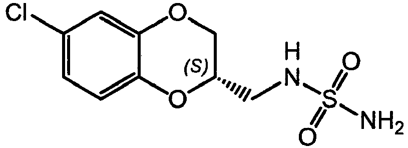

Following the procedure outlined in Example 4 above, 4-chlorocatechol was reacted to yield a mixture of (2S)-C-(7-Chloro-2,3-dihydro-benzo[1,4]dioxin-2-yl)-methylamine and (2S)-C-(6-Chloro-2,3-dihydro-benzo[1,4]dioxin-2-yl)-methylamine (ca. 3:1 ratio of 6-chloro:7-chloro isomers by RP HPLC).

The mixture was dissolved in 2-propanol (100 mL) and 1 N HCl in diethyl ether was added until pH = 1.0 was attained. The hydrochloride salt that precipitated was filtered (2.65 g) and re-crystallized from methanol/IPA to yield white crystals. The white crystals were partitioned between DCM and dilute NaOH. The DCM was dried and evaporated in vacuo to yield purified (2S)-C-(6-Chloro-2,3-dihydro-benzo[1,4]dioxin-2-yl)-methylamine as an oil.
[α]_{D} = -67.8 (c = 1.51, CHCl₃)

The oil (7.75 mmol) and sulfamide (1.50 g, 15.5 mmol) were combined in dioxane (50 mL) and refluxed for 2.0 h, cooled to room temperature and evaporated in vacuo to yield a solid. The product was purified via flash column using DCM/methanol 20:1 to yield the title compound as a white solid.
MS 277 (M⁻¹)
[α]_{D} = -59.9° (c = 1.11, M)
¹H NMR (CDCl₃) δ 6.90 (d, J = 2.2 Hz, 1 H), 6.81 (m, 2H), 4.76 (m, 1 H), 4.55 (s, 2H), 4.40 (m, 1 H), 4.29 (dd, J = 2.4, 11.5 Hz, 1 H), 4.05 (dd, J = 7.1, 11.5 Hz, 1 H), 3.45 (m, 2H)
Elemental Analysis:

| | |
|---|---|
| Anal Calc: | C, 38.78; H, 3.98; N, 10.05 |
| Anal Found: | C, 38.80; H, 3.67; N, 9.99. |

The filtrates of the crystallized hydrochloride salt of (2S)-C-(6-Chloro-2,3-dihydro-benzo[1,4]dioxin-2-yl)-methylamine prepared above were recovered (ca. 1:1 of 6-chloro:7-chloro isomers) and evaporated in vacuo to yield a solid, which was partitioned between DCM (200 mL) and dilute NaOH (0.5 M, 50 mL). The DCM solution was washed once with brine, dried (Na₂SO₄) and evaporated in vacuo to yield an oil, which was purified via reverse phase HPLC (10 - 50% ACN with 0.16% TFA in water with 0.20% TFA) to yield (2S)-C-(7-Chloro-2,3-dihydro-benzo[1,4]dioxin-2-yl)-methylamine as a residue.

The residue was combined with sulfamide (0.90 g, 9.4 mmol) in dioxane (25 mL) and refluxed for 2.5 h, cooled to room temperature and evaporated in vacuo to yield an oil. The oil was purified by flash column chromatography using DCM/methanol - 10:1 to yield (2S)-(-)-N-(7-Chloro-2,3-dihydro-benzo[1,4]dioxin-2-ylmethyl)-sulfamide as a white solid.
MS 277 (M⁻¹)
¹H NMR (CDCl₃/CD₃OD) δ 6.88 (d, J = 0.7 Hz, 1H), 6.81 (m, 2H), 4.37 (m, 1 H), 4.30 (dd, J = 2.3, 11.6 Hz, 1 H), 4.04 (dd, J = 7.0, 11.6 Hz, 1 H), 3.38 (m, 2H).

### Example 8

### Chroman-2-ylmethylsulfamide (Compound #10)

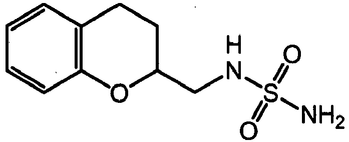

Chroman-2-carboxylic acid (4.5 g, 25 mmol) and HOBT (3.86 g, 25 mmol) were combined in DCM (40 mL) and DMF (10 mL). Dimethylaminopropyl ethylcarbodiimide (EDC, 4.84 g, 25 mmol) was added at room temperature and the reaction mixture was stirred for 30 min. Ammonium hydroxide (2.26 mL, 33.4 mmol) was added and the reaction mixture was stirred for 16h. The reaction mixture was diluted with DCM (50 mL) and water (50 mL) and the pH of the mixture was adjusted to about pH = 3.0 with 1 N HCl. The DCM was separated and the aqueous phase extracted twice with DCM. The combined DCM phase was dried (Na₂SO₄) and evaporated in vacuo to yield an oil, which was purified with flash column chromatography (ethyl acetate) to yield an oil.

The oil (5.35 g, 30 mmol) in THF (90 mL) was stirred as 1 M LAH in THF (36 mL, 36 mmol) was added and the reaction mixture was then stirred at room temperature for 20 h. The reaction was quenched with water, stirred for 2 hours, the solution decanted, dried (Na₂SO₄) and evaporated in vacuo to yield C-chroman-2-yl-methylamine as an oily amine.

The oily amine (1.63 g, 10 mmol) and sulfamide (1.92 g, 20 mmol) were combined in dioxane (50 mL) and brought to reflux for 2 h. The solution was cooled and evaporated in vacuo to yield an oil, which was purified via column chromatography (DCM:Methanol 10:1) to yield a white solid. The solid was recrystallized from ethyl acetate/hexane to yield chroman-2-ylmethylsulfamide as a white solid.
mp 100-101°C
MS 241 (M⁻¹)
Elemental Analysis:

| | |
|---|---|
| Anal Calc: | C, 49.57; H, 5.82; N, 11.56; S, 13.23 |
| Anal Found: | C, 49.57; H, 5.80; N, 11.75; S, 13.33. |

### Example 9

### 2-(2,3-Dihydro-benzo[1,4]dioxin-2-yl)-ethylsulfamide (Compound #16)

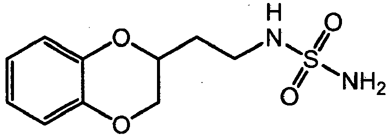

Potassium cyanide (2.05 g, 31.5 mmol) was added to 2-bromomethyl-(2,3 dihydrobenzo[1,4]dioxine) (6.87 g, 30 mmol) in DMSO (90 mL) and stirred at ambient temperature for 20 h. The reaction mixture was then diluted with water (250 mL) and extracted twice with diethyl ether. The diethyl ether was washed with water, then washed twice with brine, dried (Na₂SO₄) and evaporated in vacuo to yield 2-cyanomethyl-(2,3 dihydrobenzo[1,4]dioxine) as a white solid.
¹H NMR (CDCl₃) δ 6.89 (m, 4H), 4.50 (m, 1 H), 4.31 (dd, J = 2.3, 11.5 Hz, 1 H), 4.08 (dd, J = 6.2, 11.6 Hz, 1 H), 2.78 (d, J = 6.1, Hz, 2H)

The 2-cyanomethyl-(2,3 dihydrobenzo[1,4]dioxine) was dissolved in THF (50 mL) and 1 M BH₃ in THF (80 mL, 80 mmol) was added and the reaction mixture refluxed for 5 h, then stirred at ambient temperature for 16h. With ice bath cooling, 2N HCl was added until pH = 1.0 was achieved. The reaction mixture was then stirred for 1 h at room temperature and evaporated in vacuo to yield an oil. The oil was partitioned between 3N NaOH and diethyl ether, and the diethyl ether solution was washed with brine, dried (Na₂SO₄) and evaporated in vacuo to yield crude 2-(2,3 dihydrobenzo[1,4]dioxin-2-yl)ethylamine.
MS (M+H)⁺ 180.

The crude 2-(2,3 dihydrobenzo[1,4]dioxin-2-yl)ethylamine in dioxane (100 mL) was combined with sulfamide (3.0 g, 31 mmol) and heated to reflux for 2 h. The solution was cooled and evaporated in vacuo to yield an orange solid, which was purified by column chromatography (DCM:MeOH - 10:1) to yield a white solid. The solid was re-crystallized from DCM to yield the title compound as a solid.
MS (M-1) 257
MP 101 - 103°C (corr)
¹H NMR (CDCl₃): δ 6.86 (m, 4H), 4.70 (m, 1H), 4.52 (s, 2H), 4.30 (m, 2H), 3.94 (dd, J = 7.4, 11.3 Hz, 1 H), 3.43 (dd, J = 6.4, 12.9 Hz, 2H), 1.94 (dd, J = 6.5, 12.9, 2H).
Elemental Analysis:

| | |
|---|---|
| Measured: | C, 46.48; H, 5.60; N, 10.81; S, 12.41 |
| Calculated: | C, 46.50; H, 5.46; N, 10.85; S, 12.41 |

### Example 10

### (2S)-(-)-N-(6,7 Dichloro-2,3-dihydro-benzo[1,4]dioxin-2-ylmethyl)-sulfamide (Compound #29)

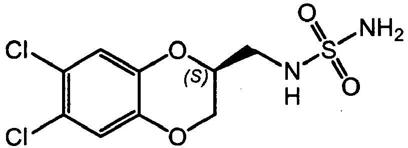

4,5 Dichloroatechol (8.6 g, 48 mmol) and potassium carbonate (6.64 g, 48 mmol) were stirred in DMF (200 mL). (2R)-Glycidyl tosylate (9.12 g, 40 mmol) was added and the reaction mixture was stirred at 60°C for 24 h. The reaction mixture was cooled to room temperature and then diluted with ice water (600 mL) and extracted with diethyl ether (4 times). The combined organic solution was washed 3 times with 10% potassium carbonate, twice with brine, dried (MgSO₄) and evaporated in vacuo to yield a viscous oil of (2S)-2-(6,7-dichloro-2,3-dihydro-benzo[1,4]dioxine) methanol.

The (2S)-2-(6,7 dichloro-2,3-dihydro-benzo[1,4]dioxine) methanol oil (6.4 g, 27 mmol) was dissolved in pyridine (50 mL) cooled to 0°C. Then, p-toluenesulfonyl chloride (5.2 g, 27 mmol) was added and the reaction mixture was stirred at room temperature for 20h. The reaction mixture was diluted with diethyl ether and 1 N HCl (750 mL) and the organic layer was separated and washed 2 times with 1 N HCl (250 mL), once with water (150 mL), twice with brine, dried (MgSO₄) and evaporated in vacuo to yield light yellow solid of toluene-4-sulfonic acid (2S)-6,7-dichloro-2,3-dihydro-benzo[1,4]dioxin-2-ylmethyl ester.
¹H NMR (CDCl₃): δ 7.79 (d, J = 8.3 Hz, 2H), 7.36 (d, J = 8.0 Hz, 2H), 6.94 (s, 1H), 6.83 (s, 1 H), 4.37 (m, 1 H), 4.2 (m, 3H), 4.03 (dd, J = 6.3, 11.7 Hz, 1H), 2.47 (s, 3H).

Toluene-4-sulfonic acid (2S)-6,7-dichloro-2,3-dihydro-benzo[1,4]dioxin-2-ylmethyl ester (8.0 g, 20.5 mmol) was combined with potassium phthalimide (6.1 g, 33 mmol) in DMF (75 mL) and heated to reflux for 1 h, cooled to room temperature and poured into vigorously stirring water (0.5 L) and then stirred 30 min. White solid was filtered and the solid was washed several times with water, 2% NaOH, and water again and then let air dry to yield (2S)-2-(6,7-dichloro-2,3-dihydro-benzo[1,4]dioxin-2-ylmethyl)-isoindole-1,3-dione (6.0 g, 80%) as a white powdery solid.

The white powdery solid was combined with hydrazine (1.06 g, 33 mmol) in EtOH (80 mL) and heated at reflux for 2 h, then cooled to room temperature. 1 N HCl was added to adjust the reaction mixture's pH to pH 1.0 and the reaction mixture was then stirred for 15 min. White solid was filtered and washed with fresh EtOH (solid discarded) and the filtrate was evaporated in vacuo to a solid, which was partitioned between diethyl ether and dilute aqueous NaOH. The diethyl ether solution was dried (Na₂SO₄) and evaporated in vacuo to a yield a viscous oil of (2S)-2-aminomethyl-(6,7-dichloro-2,3-dihydro-benzo[1,4]dioxine).
¹H NMR (CDCl₃): δ 6.98 (s, 1 H), 6.96 (s, 1H), 4.25 (dd, J = 2.0,11.2 Hz, 1 H), 4.15 (m, 1H), 4.0 (m, 1 H), 2.97 (d, J = 5.5 Hz, 2H)

A portion of the oil (3.8 g, 16 mmol) and sulfamide (3.1 g, 32.4 mmol) were refluxed in dioxane (100 mL) for 2 h and the crude product was purified by flash column chromatography (DCM:MeOH 20:1) to yield the title compound as a white solid, which was recrystallized from ethyl acetate / hexane to yield the title compound as a white crystalline solid.
MS [M-H]⁻ 311.0
mp 119-121°C
[α]_{D}=-53.4° (c=1.17, M)
¹H NMR (DMSOd6): δ 7.22 (s, 1H), 7.20 (s, 1H), 6.91 (bd s, 1H), 6.68 (bd s, 2H), 4.35 (m, 2H), 4.05 (dd, J = 6.5, 11.5 Hz, 1 H), 3.15 (m, 2H) Elemental Analysis:
Elemental Analysis:

| | |
|---|---|
| Measured: | C, 34.52; H, 3.22; N, 8.95; Cl, 22.64; S, 10.24 |
| Calculated: | C, 34.64; H, 2.68; N, 8.87; Cl, 22.94; S, 10.35. |

### Example 11

### (2S)-(-)-N-(7-Amino-2,3-dihydro-benzo[1,4]dioxin-2-ylmethyl)-sulfamide (Compound #36)

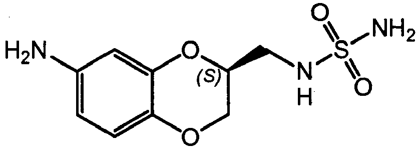

(2S)-(-)-N-(2,3-Dihydro-7-nitro-benzo[1,4]dioxin-2-ylmethylysulfamide (1.2 g, 4.15 mmol), was prepared from 4-nitrocatechol according to the process outlined in Example 4. The (2S)-(-)-N-(2,3-Dihydro-7-nitro-benzo[1,4]dioxin-2-ylmethyl)-sulfamide, was then combined with 10% Pd/C in methanol (120 mL) and shaken under hydrogen atmosphere (39 psi) at room temperature for 3 h. The solids were filtered and washed with 10% M in DCM and the filtrate was evaporated in vacuo to yield crude product. The crude product was dissolved in 0.2 N HCl (25 mL), frozen and lyophilized to yield the title compound as a white flaky solid, as the corresponding hydrochloride salt.
MS (M+H)⁺ 260
¹H NMR (DMSO d6): δ 10.2 (bd s, 3H), 6.86 (m, 1 H), 6.85 (s, 1H), 6.74 (dd, J = 2.5, 8.4 Hz, 1 H), 4.22 (m, 2H), 3.88 (dd, J = 6.7, 11.4 Hz, 1 H), 3.04 (m, 2H)

### Example 12

### (2S)-(-)-N-(7-Methyl-2,3-dihydrobenzo[1,4]dioxin-2-ylmethyl)-sulfamide (Compound #19)

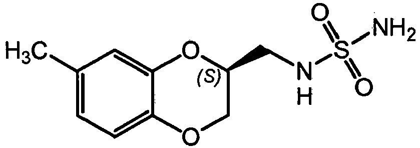

Title compound was prepared according to the procedure described in Example 4 above, starting with 4-methylcatechol, to yield a white solid, which was recrystallized from ethyl acetate/ hexane to yield the title compound as a white solid.
MS [M-H]⁻257
¹H NMR (CDCl₃): δ 6.76 (m, 1 H), 6.66 (m, 2H), 4.80 (m, 1 H), 4.57 (bd s, 1 H), 4.40 (m, 1H), 4.28 (m, 1 H), 4.03 (dd, J = 6.9, 11.4 Hz, 1 H), 3.45 (m, 2H), 2.25 (s, 3H).
Elemental Analysis

| | |
|---|---|
| Calculated: | C, 46.50; H, 5.46; N, 10.85; S, 12.41 |
| Found: | C, 46.65; H, 5.60; N, 10.84; S, 12.61 |

### Example 13

### Elevated Plus Maze, Mouse (EPM)

The EPM *in vivo* assay is used to determine if a test compound possesses anxiolytic (anti-anxiety) activity. Rodents avoid open spaces (in the case of the assay apparatus, the open arms of an elevated plus-maze). Compounds which have anti-anxiety activity increase exploratory activity of the rodents in the open arms of the elevated plus-maze. The EPM assay was run according to the procedure as described by Handley and Mithani (Naunyn. Schmied. Arch. Pharmacol., 327, 1-5, 1984*).*

Male Rj:NMRI mice, weighing 20 - 30 g (max. range per experiment = 5 g) at the beginning of the experiments, were obtained from Elevage Janvier, 53940 Le Genest-Saint-Isle, France and used in the assay. The animals were delivered to the laboratory at least 5 days before the experiments during which time they were acclimatized to laboratory conditions. The mice were housed in groups of 10 in Macrolon cages (25 x 19 x 13 cm) on wood litter (Litalabo-SPPS, 95100 Argenteuil, France) with free access to food (Code 113 - SAFE, 89290 Augy, France) and water until tested (or as indicated otherwise). The animal house was maintained under artificial lighting (12 hours) between 7:00 and 19:00 in a controlled ambient temperature of 21 ± 3°C, and relative humidity between 30-80%.

The elevated plus-maze consisted of 4 arms of equal length and width (14 x 5 cm) arranged in the form of a plus sign (+). Two opposite arms were enclosed by 12 cm high walls (closed arms). The 2 other arms had no walls (open arms). The maze was raised 56 cm above the floor. A mouse was placed in the centre of the plus-maze and left to explore the maze for 5 minutes. The number of entries into the open and closed arms and the time spent in the open arms was recorded. Ten (10) mice were used for each study group. The test was performed blind.

Compound #8 was evaluated at 30 mg/kg, 60 mg/kg and 120 mg/kg doses, administered p.o. 60 minutes before the test, and compared with a vehicle control group. Compound #8 was homogenized and dispersed using a mortar and a pestle in 0.2% hydroxypropylmethylcellulose (HPMC) in distilled water, which served as the vehicle. As a positive control, Clobazam (16 mg/kg p.o.), was administered under the same experimental conditions. Clobazam as URBANYL^{®}tablets (16 mg/kg p.o.) were dispersed in 0.2% hydroxypropylmethylcellulose (HPMC) in distilled water for administration. Each experiment therefore included 5 study groups of 10 mice each.

Data were analyzed by comparing groups treated with compound #8 with groups treated with vehicle control using unpaired Student's t tests. The results (including mean time, % change from control and Student's t-test p value) are listed in Table 4, below.

**Table 4: Effect of Compound #8 in EPM**

| | **Open Arms Number of Entries** | | **Open Arms Time Spent (sec)** | | **Closed Arms Number of Entries** | |
|---|---|---|---|---|---|---|
| | **Mean ± S.E.M.** | **% change** | **Mean ± S.E.M.** | **% change** | **Mean ± S.E.M.** | **% change** |
| Vehicle | 2.6 ± 0.4 | - | 18.0 ± 2.9 | - | 7.5 ± 0.5 | - |
| Cmpd #8 30 mg/kg | 3.9 ±0.7 (NS) | +50% | 44.6 ± 8.0 (**) | +148% | 8.5 ± 0.9 (NS) | +13% |
| Cmpd #8 60 mg/kg | 5.9 ± 1.0 (**) | +127% | 54.1 ± 10.6 (**) | +201% | 10.3 ± 0.8 (**) | +37% |
| Cmpd #8 120 mg/kg | 4.5 ± 0.6 (*) | +73% | 38.6 ± 5.2 (**) | +114% | 10.0 ± 1.1 (*) | +33% |
| CLOBAZAM 16 mg/kg | 11.7 ± 1.1 (***) | +350% | 120.1 ± 13.1 (***) | +567% | 11.9 ± 1.5 (*) | +59% |

| | | | | | | |
|---|---|---|---|---|---|---|
| Student's t test: (NS) = Not Statistically Significant; (*) = p < 0.05; (**) = p < 0.01; (***) = p < 0.001 | | | | | | |

### Example 14

### Four Plate Test, Mouse

The Four Plate *in vivo* assay is used to determine if a test compound possesses anxiolytic (anti-anxiety) activity. The Four Plate assay was run according to the procedure as described by Aron et al (Neuropharmacology, 10, 459-469, 1971*).* In this assay, the rodent experiences an electric shock upon crossing from one plate to another. Anxiolytics (such as benzodiazepines) increase the number of punished crossings.

Animals were placed individually in a white plastic enclosure containing a floor consisting of 4 metal plates connected to an electric shock generator (Apelex : Type 011346). The mouse was left to explore freely for 15 seconds. Then, every time the mouse crossed from one plate to another, it received a weak electric shock (2.5 mA, 1.5 s). The number of punished crossings were counted during a 1 minute test. Ten (10) mice were used for each study group. The test was performed blind.

Compound #8 was evaluated at 30 mg/kg, 60 mg/kg and 120 mg/kg doses, administered p.o. 60 minutes before the test, and compared with a vehicle control group. Compound #8 was homogenized and dispersed using a mortar and a pestle in 0.2% hydroxypropylmethylcellulose (HPMC) in distilled water, which served as the vehicle. As a positive control, Clobazam (32 mg/kg p.o.), was administered under the same experimental conditions. Clobazam as URBANYL^{®}tablets (32 mg/kg p.o.) were dispersed in 0.2% hydroxypropylmethylcellulose (HPMC) in distilled water for administration. Each experiment therefore included 5 study groups of 10 mice each.

Data were analyzed by comparing groups treated with compound #8 with groups treated with vehicle control using unpaired Student's t tests. The results (including mean number of crossing, % change from control and Student's t-test p value) are listed in Table 5, below.

**Table 5: Effect of Compound #8 in Four Plate**

| | **Number of Punished Crossings** | |
|---|---|---|
| | **Mean ± S.E.M.** | **% Change** |
| Vehicle | 3.9 ± 0.6 | - |
| Cmpd #8 30 mg/kg | 4.5 ± 0.6 (NS) | +15% |
| Cmpd #8 60 mg/kg | 4.5 ± 0.3 (NS) | +15% |
| Cmpd #8 120 mg/kg | 6.6 ± 0.6 (**) | +69% |
| CLOBAZAM 32 mg/kg | 9.9 ± 1.2 (***) | + 154% |

| | | |
|---|---|---|
| Student's t test: (NS) = Not Significant; (**) = p < 0.01; (***) = p < 0.001 | | |

### Example 15

As a specific embodiment of an oral composition, 100 mg of the Compound #8 prepared as in Example 7 is formulated with sufficient finely divided lactose to provide a total amount of 580 to 590 mg to fill a size O hard gel capsule.

While the foregoing specification teaches the principles of the present invention, with examples provided for the purpose of illustration, it will be understood that the practice of the invention encompasses all of the usual variations, adaptations and/or modifications as come within the scope of the following claims.

## Claims

1. A compound of formula (I) or a pharmaceutically acceptable salt thereof, for use in a method of treating anxiety or a related disorder, wherein the anxiety or related disorder is selected from the group consisting of generalized anxiety disorder, acute stress disorder, post traumatic stress disorder, obsessive-compulsive disorder, social phobia (also known as social anxiety disorder), specific phobia, panic disorder with or without agoraphobia, agoraphobia without a history of panic disorder and substance abuse induced anxiety disorder 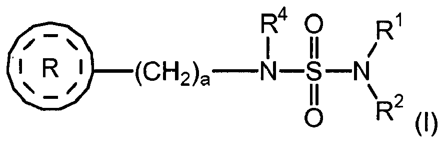 wherein
R¹ and R² are each independently selected from the group consisting of hydrogen and lower alkyl;
R⁴ is selected from the group consisting of hydrogen and lower alkyl;
a is an integer from 1 to 2; 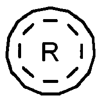 is selected from the group consisting of 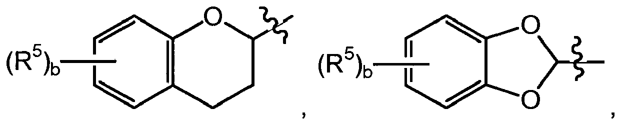 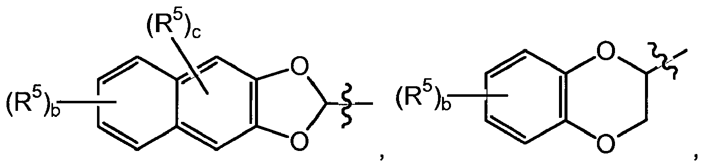 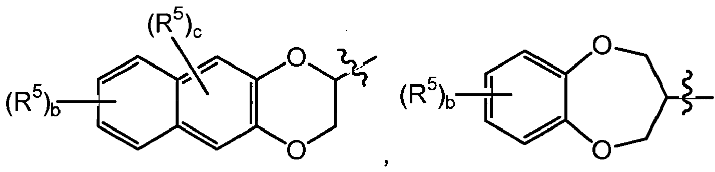 and 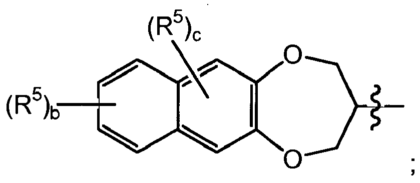
wherein b is an integer from 0 to 4; and wherein c is an integer from 0 to 2;
each R⁵ is independently selected from the group consisting of halogen and lower alkyl;
provided that when 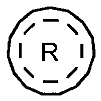 is 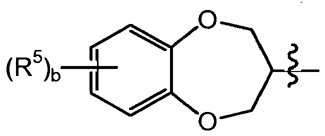 or 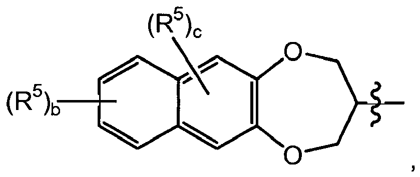 then a is 1;
wherein "lower" when used with "alkyl" means a carbon chain composition of 1-4 carbon atoms.

2. The compound or pharmaceutically acceptable salt thereof for use as claimed in Claim 1, wherein
R¹ and R² are each independently selected from the group consisting of hydrogen and lower alkyl;
R⁴ is selected from the group consisting of hydrogen and lower alkyl; a is an integer from 1 to 2; 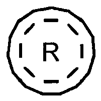 is selected from the group consisting of 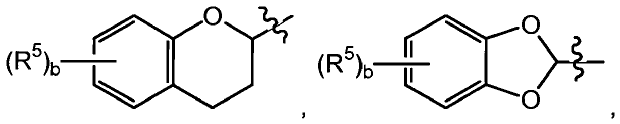 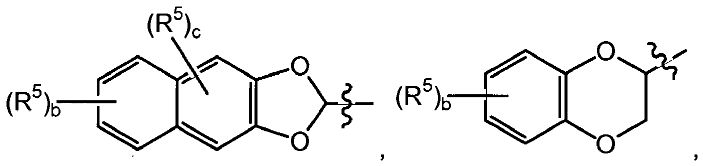 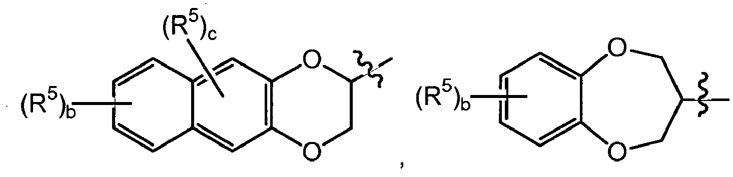 and 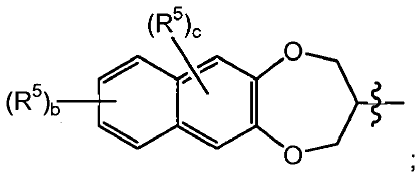 wherein b is an integer from 0 to 2; and wherein c is an integer from 0 to 1;
each R⁵ is independently selected from the group consisting of halogen and lower alkyl;
provided that when 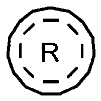 is 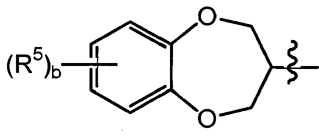 or 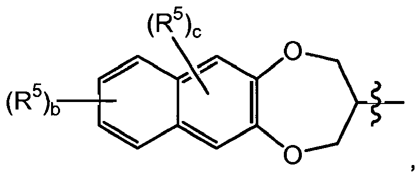 then a is 1.

3. The compound or pharmaceutically acceptable salt thereof for use as claimed in Claim 2, wherein
R¹ and R² are each independently selected from the group consisting of hydrogen and lower alkyl;
R⁴ is selected from the group consisting of hydrogen and lower alkyl; a is an integer from 1 to 2; 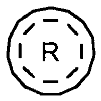 is selected from the group consisting of 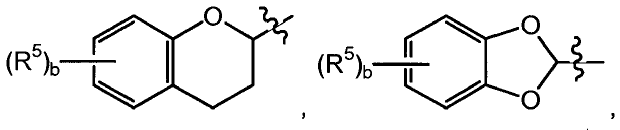 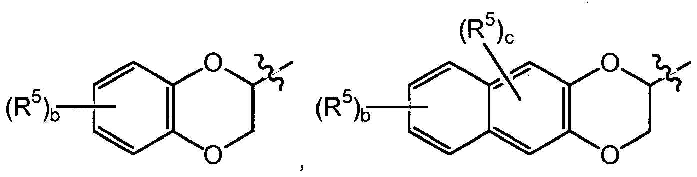 and 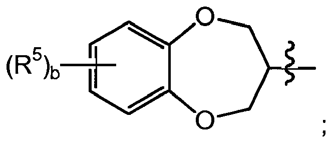 wherein b is an integer from 0 to 2; and wherein c is 0;
each R⁵ is independently selected from the group consisting of halogen and lower alkyl;
provided that when 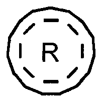 is 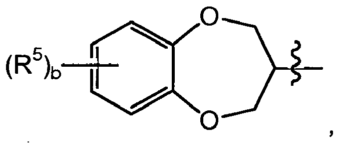 then a is 1.

4. The compound or pharmaceutically acceptable salt thereof for use as claimed in Claim 3, wherein
R¹ and R² are each independently selected from the group consisting of hydrogen and lower alkyl;
R⁴ is selected from the group consisting of hydrogen and methyl;
a is an integer from 1 to 2; 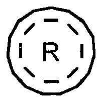 is selected from the group consisting of 2-(2,3-dihydro-benzo[1,4]dioxinyl), 2-(benzo[1,3]dioxolyl), 2-(3,4-dihydro-2H-benzo[1,4]dioxepinyl), 2-(6-chloro-2,3-dihydro-benzo[1,4]dioxinyl), 2-(6-fluoro-2,3-dihydro-benzo[1,4]dioxinyl), 2-(chromanyl), 2-(5-fluoro-2,3-dihydro-benzo[1,4]dioxinyl), 2-(7-chloro-2,3-dihydro-benzo[1,4]dioxinyl), 2-(6-chloro-benzo[1,3]dioxolyl), 2-(7-methyl-2,3-dihydro-benzo[1,4]dioxinyl), 2-(5-chloro-2,3-dihydro-benzo[1,4]dioxinyl), 2-(6-bromo-2,3-dihydro-benzo[1,4]dioxinyl), 2-(6,7-dichloro-2,3-dihydro-benzo[1,4]dioxinyl), 2-(8-chloro-2,3-dihydro-benzo[1,4]dioxinyl), 2-(2,3-dihydro-naphtho[2,3-b][1,4]dioxinyl) and 2-(4-methyl-benzo[1,3]dioxolyl);
provided that when 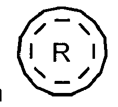 is 2-(3,4-dihydro-2H-benzo[1,4]dioxepinyl), then a is 1.

5. The compound or pharmaceutically acceptable salt thereof for use as claimed in Claim 4, wherein
R¹ and R² are each independently selected from the group consisting of hydrogen and methyl;
R⁴ is selected from the group consisting of hydrogen and methyl;
a is an integer from 1 to 2; 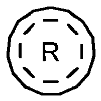 is selected from the group consisting of 2-(benzo[1,3]dioxolyl), 2-(2,3-dihydro-benzo[1,4]dioxinyl), 2-(6-chloro-2,3-dihydro-benzo[1,4]dioxinyl), 2-(7-chloro-2,3-dihydro-benzo[1,4]dioxinyl), 2-(7-methyl-2,3-dihydro-benzo[1,4]dioxinyl), 2-(6-bromo-2,3-dihydro-benzo[1,4]dioxinyl) and 2-(6,7-dichloro-2,3-dihydro-benzo[1,4]dioxinyl).

6. A compound selected from the group consisting (2S)-(-)-N-(6-chloro-2,3-dihydro-benzo[1,4]dioxin-2-ylmethyl)-sulfamide; and pharmaceutically acceptable salts thereof for use in a method of treating anxiety or a related disorder, wherein the anxiety or related disorder is selected from the group consisting of generalized anxiety disorder, acute stress disorder, post traumatic stress disorder, obsessive-compulsive disorder, social phobia (also known as social anxiety disorder), specific phobia, panic disorder with or without agoraphobia, agoraphobia without a history of panic disorder and substance abuse induced anxiety disorder.

7. A compound of formula (III) or a pharmaceutically acceptable salt thereof for use in the treatment of anxiety or a related disorder, wherein the anxiety or related disorder is selected from the group consisting of generalized anxiety disorder, acute stress disorder, post traumatic stress disorder, obsessive-compulsive disorder, social phobia (also known as social anxiety disorder), specific phobia, panic disorder with or without agoraphobia, agoraphobia without a history of panic disorder and substance abuse induced anxiety disorder

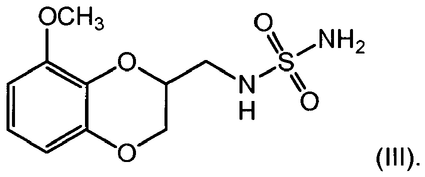

8. At least one anxiolytic and a compound of formula (I) or a pharmaceutically acceptable salt thereof as a combined preparation for simultaneous, separate or sequential use in a method for the treatment of anxiety or a related disorder, wherein the anxiety or related disorder is selected from the group consisting of generalized anxiety disorder, acute stress disorder, post traumatic stress disorder, obsessive-compulsive disorder, social phobia (also known as social anxiety disorder), specific phobia, panic disorder with or without agoraphobia, agoraphobia without a history of panic disorder and substance abuse induced anxiety disorder 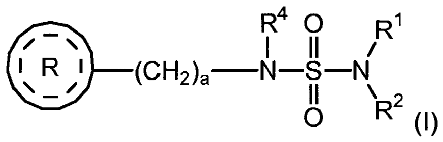 wherein
R¹ and R² are each independently selected from the group consisting of hydrogen and lower alkyl;
R⁴ is selected from the group consisting of hydrogen and lower alkyl; a is an integer from 1 to 2; 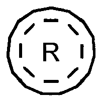 is selected from the group consisting of 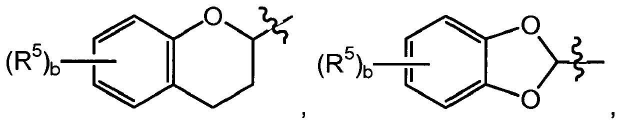 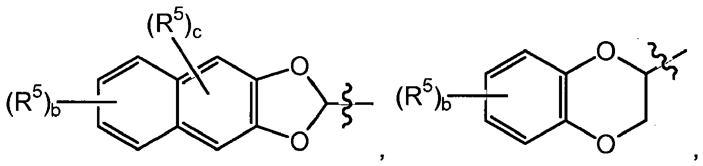 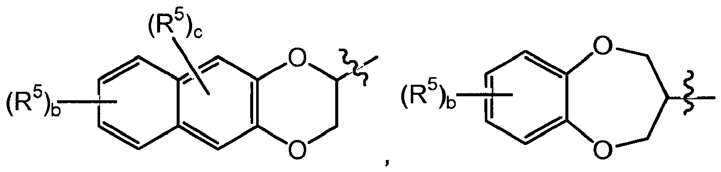 and 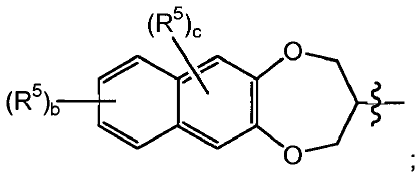
wherein b is an integer from 0 to 4; and wherein c is an integer from 0 to 2;
each R⁵ is independently selected from the group consisting of halogen and lower alkyl;
provided that when 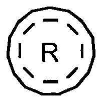 is 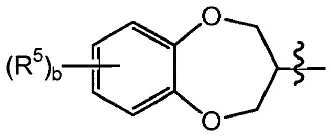 or 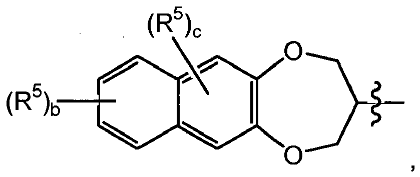 then a is 1;
wherein "lower" when used with "alkyl" means a carbon chain composition of 1-4 carbon atoms.

9. The compound or pharmaceutically acceptable salt thereof for use as claimed in Claim 1, or the at least one anxiolytic and a compound of formula (I) or a pharmaceutically acceptable salt thereof as a combined preparation for use as claimed in claim 8, wherein the compound of formula (I) is selected from the group consisting of (2S)-(-)-N-(6-chloro-2,3-dihydro-benzo[1,4]dioxin-2-ylmethyl)-sulfamide; and pharmaceutically acceptable salts thereof.

10. At least one anxiolytic and a compound selected from the group consisting of (2S)-(-)-N-(6-chloro-2,3-dihydro-benzo[1,4]dioxin-2-ylmethyl)-sulfamide; and pharmaceutically acceptable salts thereof as a combined preparation for simultaneous, separate or sequential use in a method for the treatment of anxiety or a related disorder, wherein the anxiety or related disorder is selected from the group consisting of generalized anxiety disorder, acute stress disorder, post traumatic stress disorder, obsessive-compulsive disorder, social phobia (also known as social anxiety disorder), specific phobia, panic disorder with or without agoraphobia, agoraphobia without a history of panic disorder and substance abuse induced anxiety disorder.

11. The compound or pharmaceutically acceptable salt thereof for use as claimed in any one of Claims 1-7 and 9, or the at least one anxiolytic and a compound or a pharmaceutically acceptable salt thereof as a combined preparation for use as claimed in Claim 8, 9 or 10, wherein the anxiety or related disorder is selected from the group consisting of generalized anxiety disorder, acute stress disorder, post traumatic stress disorder and obsessive-compulsive disorder.

12. The compound, pharmaceutically acceptable salt thereof for use as claimed in Claim 11, or the at least one anxiolytic and a compound or a pharmaceutically acceptable salt thereof as a combined preparation for use as claimed in Claim 11, wherein the anxiety or related disorder is generalized anxiety disorder.

13. The at least one anxiolytic and a compound or a pharmaceutically acceptable salt thereof as a combined preparation for use as claimed in Claim 8, 9 or 10, wherein the anxiolytic is selected from the group consisting of benzodiazepines, selective serotonin reuptake inhibitors, selective noradrenergic reuptake inhibitors, serotonin receptor antagonists, norepinephrine reuptake inhibitors, dopamine reuptake inhibitors, dopamine receptor antagonist, corticotropin releasing factor antagonists, monoamine oxidase inhibitors, 5HT₁ₐ agonists, Alpha2 adrenergic antagonists, GABA_{A} agonists, GABA_{B} antagonists, cannabanoid agonists, neurokinin antagonists, kappa opiod antagonists and opiod Receptor Like-1 agonists.

14. The at least one anxiolytic and a compound or a pharmaceutically acceptable salt thereof as a combined preparation for use as claimed in Claim 13, wherein the anxiolytic is selected from the group consisting of benzodiazepines, selective serotonin reuptake inhibitors (SSRIs) and selective noradrenergic reuptake inhibitors.

15. The at least one anxiolytic and a compound of formula (I) or a pharmaceutically acceptable salt thereof as a combined preparation for use as claimed in Claim 8, 9 or 10, wherein the anxiolytic is selected from the group consisting of diazepam, flurazepam, triazolam, lorazepam, alprazolam, chlordiazepam, oxazepam, temazepam, clonazepam, buspirone, fluoxetine, sertraline, paroxetine, citalopram, fluvoxamine, nefazadone, venlafaxine, milnacipran, 3-(3-amino-2-(R)-hydroxy-propyl)-1-(4-fluoro-phenyl)-8-(8-methyl-naphthalen-1-ylmethyl)-1,3,8-triazaspiro[4.5]decan-4-one, 2-(2-chloro-phenyl)-2-(S)-hydroxy-ethyl ester carbamic acid and 1-[7-(4-bromo-2,6-dimethylphenyl)-2,5-dimethyl-7H-pyrrolo[2,3-d]pyrimidin-4-yl]-4-piperidinemethanol.

16. The at least one anxiolytic and a compound or a pharmaceutically acceptable salt thereof as a combined preparation for use as claimed in Claim 15, wherein the anxiolytic is selected from the group consisting of diazepam, venlafaxine, fluoxetine and pregabalin.

17. At least one anxiolytic and a compound of formula (III) or a pharmaceutically acceptable salt thereof as a combined preparation for simultaneous, separate or sequential use in a method for the treatment of generalized anxiety disorder, acute stress disorder, post traumatic stress disorder, obsessive-compulsive disorder, social phobia (also known as social anxiety disorder), specific phobia, panic disorder with or without agoraphobia, agoraphobia without a history of panic disorder or substance abuse induced anxiety disorder

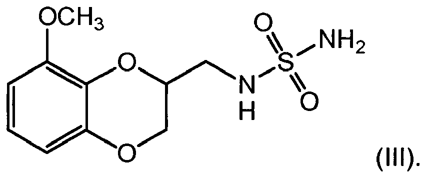

## Patentansprüche

1. Verbindung der Formel (I) oder ein pharmazeutisch unbedenkliches Salz davon zur Verwendung bei einer Methode zur Behandlung von Angst oder einer verwandten Störung, wobei die Angst oder verwandte Störung aus der Gruppe bestehend aus generalisierter Angstneurose, akuter Stresserkrankung, posttraumatischer Stresserkrankung, Zwangsneurose, sozialer Phobie (auch bekannt als soziale Angstneurose), spezifischer Phobie, Panikerkrankung mit oder ohne Agoraphobie, Agoraphobie ohne Vorgeschichte von Panikerkrankung und durch Substanzmissbrauch induzierter Angstneurose 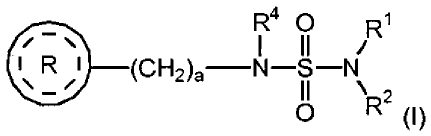 wobei
R¹ und R² jeweils unabhängig voneinander aus der Gruppe bestehend aus Wasserstoff und Niederalkyl ausgewählt sind;
R⁴ aus der Gruppe bestehend aus Wasserstoff und Niederalkyl ausgewählt ist;
a für eine ganze Zahl von 1 bis 2 steht; 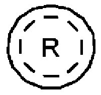 aus der Gruppe bestehend aus 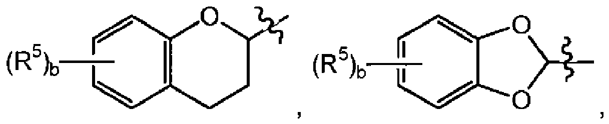 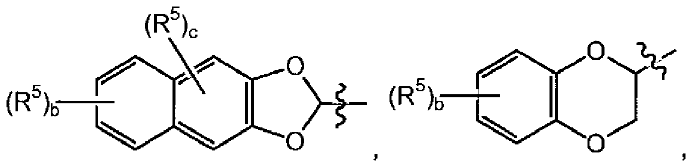 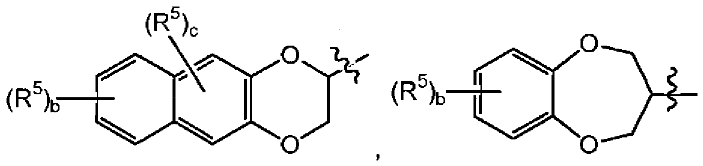 und 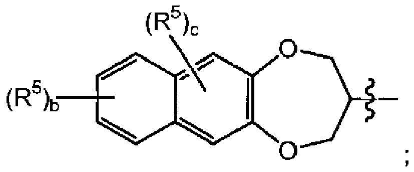
ausgewählt ist, wobei b für eine ganze Zahl von 0 bis 4 steht und c für eine ganze Zahl von 0 bis 2 steht;
R⁵ jeweils unabhängig voneinander aus der Gruppe bestehend aus Halogen und Niederalkyl ausgewählt ist;
mit der Maßgabe, dass dann, wenn 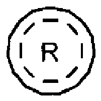 für 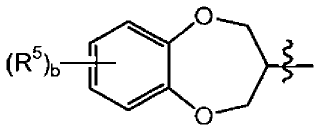 oder 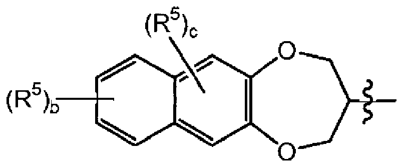 steht, a für 1 steht;
wobei "Nieder" bei Verwendung mit "Alkyl" eine Kohlenstoffkettenzusammensetzung mit 1-4 Kohlenstoffatomen bedeutet.

2. Verbindung oder pharmazeutisch unbedenkliches Salz davon zur Verwendung nach Anspruch 1, wobei
R¹ und R² jeweils unabhängig voneinander aus der Gruppe bestehend aus Wasserstoff und Niederalkyl ausgewählt sind;
R⁴ aus der Gruppe bestehend aus Wasserstoff und Niederalkyl ausgewählt ist;
a für eine ganze Zahl von 1 bis 2 steht; 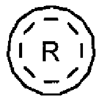 aus der Gruppe bestehend aus 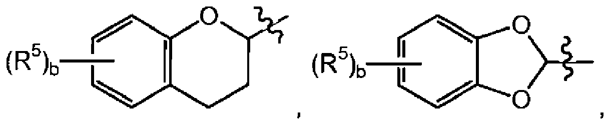 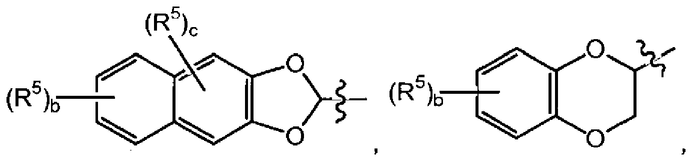 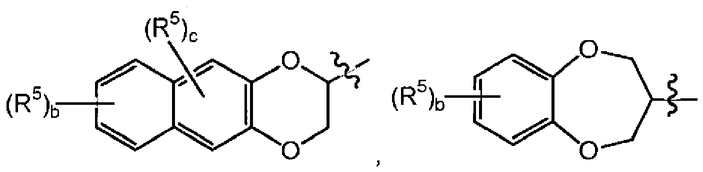 und 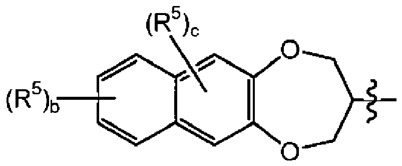 ausgewählt ist, wobei b für eine ganze Zahl von 0 bis 2 steht und c für eine ganze Zahl von 0 bis 1 steht;
R⁵ jeweils unabhängig voneinander aus der Gruppe bestehend aus Halogen und Niederalkyl ausgewählt ist;
mit der Maßgabe, dass dann, wenn 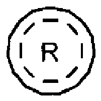 für 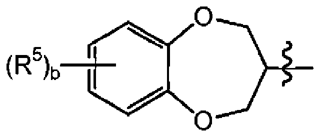 oder 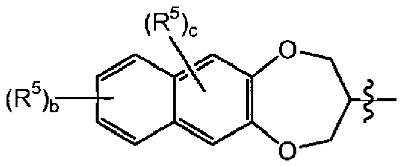 steht, a für 1 steht.

3. Verbindung oder pharmazeutisch unbedenkliches Salz davon zur Verwendung nach Anspruch 2, wobei R¹ und R² jeweils unabhängig voneinander aus der Gruppe bestehend aus Wasserstoff und Niederalkyl ausgewählt sind;
R⁴ aus der Gruppe bestehend aus Wasserstoff und Niederalkyl ausgewählt ist;
a für eine ganze Zahl von 1 bis 2 steht; 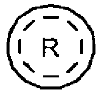 aus der Gruppe bestehend aus 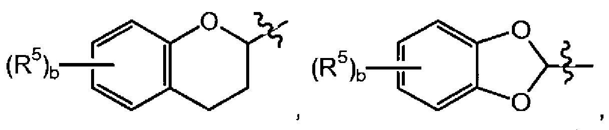 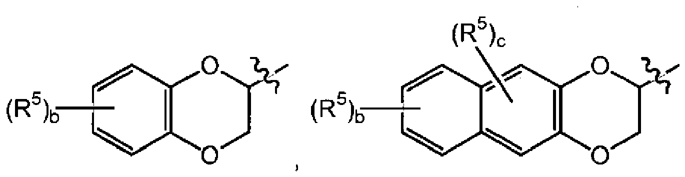 und 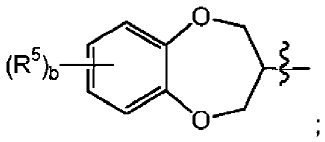 ausgewählt ist, wobei b für eine ganze Zahl von 0 bis 2 steht und c für 0 steht;
R⁵ jeweils unabhängig voneinander aus der Gruppe bestehend aus Halogen und Niederalkyl ausgewählt ist;
mit der Maßgabe, dass dann, wenn 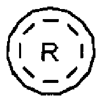 für 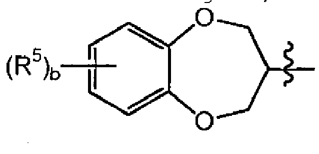 steht, a für 1 steht.

4. Verbindung oder pharmazeutisch unbedenkliches Salz davon zur Verwendung nach Anspruch 3, wobei R¹ und R² jeweils unabhängig voneinander aus der Gruppe bestehend aus Wasserstoff und Niederalkyl ausgewählt sind;
R⁴ aus der Gruppe bestehend aus Wasserstoff und Methyl ausgewählt ist;
a für eine ganze Zahl von 1 bis 2 steht; 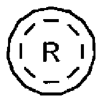 aus der Gruppe bestehend aus 2-(2,3-Dihydrobenzo[1,4]dioxinyl), 2-(Benzo[1,3]dioxolyl), 2-(3,4-Dihydro-2H-benzo[1,4]dioxepinyl), 2-(6-Chlor-2,3-dihydro-benzo[1,4]dioxinyl), 2-(6-Fluor-2,3-dihydro-benzo[1,4]dioxinyl), 2-(Chromanyl), 2-(5-Fluor-2,3-dihydro-benzo[1,4]dioxinyl), 2-(7-Chlor-2,3-dihydrobenzo[1,4]dioxinyl), 2-(6-Chlorbenzo-[1,3]dioxolyl), 2-(7-Methyl-2,3-dihydrobenzo[1,4]-dioxinyl), 2-(5-Chlor-2,3-dihydrobenzo[1,4]-dioxinyl), 2-(6-Brom-2,3-dihydrobenzo[1,4]-dioxinyl), 2-(6,7-Dichlor-2,3-dihydrobenzo[1,4]-dioxinyl), 2-(8-Chlor-2,3-dihydrobenzo[1,4]-dioxinyl), 2-(2,3-Dihydronaphtho[2,3-b][1,4]-dioxinyl) und 2-(4-Methylbenzo[1,3]dioxolyl) ausgewählt ist;
mit der Maßgabe, dass dann, wenn 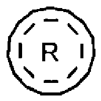 für 2-(3,4-Dihydro-2H-benzo[1,4]dioxepinyl steht, a für 1 steht.

5. Verbindung oder pharmazeutisch unbedenkliches Salz davon zur Verwendung nach Anspruch 4, wobei R¹ und R² jeweils unabhängig voneinander aus der Gruppe bestehend aus Wasserstoff und Methyl ausgewählt sind;
R⁴ aus der Gruppe bestehend aus Wasserstoff und Methyl ausgewählt ist;
a für eine ganze Zahl von 1 bis 2 steht; 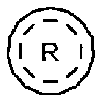 aus der Gruppe bestehend aus 2-(Benzo[1,3]dioxolyl), 2-(2,3-Dihydrobenzo[1,4]dioxinyl), 2-(6-Chlor-2,3-dihydrobenzo[1,4]dioxinyl), 2-(7-Chlor-2,3-dihydrobenzo[1,4]dioxinyl), 2-(7-Methyl-2,3-dihydrobenzo[1,4]dioxinyl), 2-(6-Brom-2,3-dihydro-benzo[1,4]dioxinyl) und 2-(6,7-Dichlor-2,3-dihydrobenzo[1,4]dioxinyl) ausgewählt ist.

6. Verbindung oder pharmazeutisch unbedenkliches Salz davon zur Verwendung nach Anspruch 1, wobei die Verbindung der Formel (I) aus der Gruppe bestehend aus (2S)-(-)-N-(6-Chlor-2,3-dihydrobenzo[1,4]-dioxin-2-ylmethyl)sulfamid und pharmazeutisch unbedenklichen Salzen davon ausgewählt ist, zur Verwendung bei einer Methode zur Behandlung von Angst oder einer verwandten Störung, wobei die Angst oder verwandte Störung aus der Gruppe bestehend aus generalisierter Angstneurose, akuter Stresserkrankung, posttraumatischer Stresserkrankung, Zwangsneurose, sozialer Phobie (auch bekannt als soziale Angstneurose), spezifischer Phobie, Panikerkrankung mit oder ohne Agoraphobie, Agoraphobie ohne Vorgeschichte von Panikerkrankung und durch Substanzmissbrauch induzierter Angstneurose ausgewählt ist.

7. Verbindung der Formel (III) oder pharmazeutisch unbedenkliches Salz davon zur Verwendung zur Behandlung von Angst oder einer verwandten Störung, wobei die Angst oder verwandte Störung aus der Gruppe bestehend aus generalisierter Angstneurose, akuter Stresserkrankung, posttraumatischer Stresserkrankung, Zwangsneurose, sozialer Phobie (auch bekannt als soziale Angstneurose), spezifischer Phobie, Panikerkrankung ohne Agoraphobie, Agoraphobie ohne Vorgeschichte von Panikerkrankung und durch Substanzmissbrauch induzierter Angstneurose 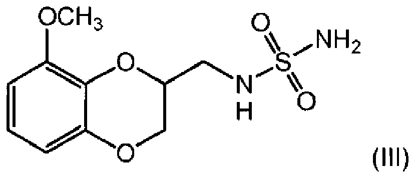 ausgewählt ist.

8. Mindestens ein Anxiolytikum und eine Verbindung der Formel (I) oder ein pharmazeutisch unbedenkliches Salz davon als Kombinationspräparat für die gleichzeitige, getrennte oder aufeinanderfolgende Verwendung bei einer Methode zur Behandlung von Angst oder einer verwandten Störung, wobei die Angst oder verwandte Störung aus der Gruppe bestehend aus generalisierter Angstneurose, akuter Stresserkrankung, posttraumatischer Stresserkrankung, Zwangsneurose, sozialer Phobie (auch bekannt als soziale Angstneurose), spezifischer Phobie, Panikerkrankung mit oder ohne Agoraphobie, Agoraphobie ohne Vorgeschichte von Panikerkrankung und durch Substanzmissbrauch induzierter Angstneurose 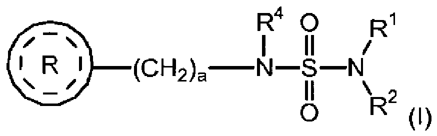 wobei
R¹ und R² jeweils unabhängig voneinander aus der Gruppe bestehend aus Wasserstoff und Niederalkyl ausgewählt sind;
R⁴ aus der Gruppe bestehend aus Wasserstoff und Niederalkyl ausgewählt ist;
a für eine ganze Zahl von 1 bis 2 steht; 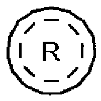 aus der Gruppe bestehend aus 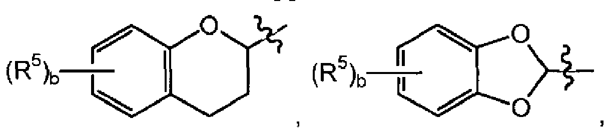 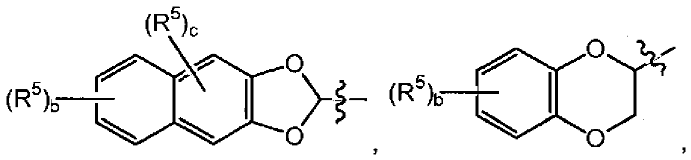 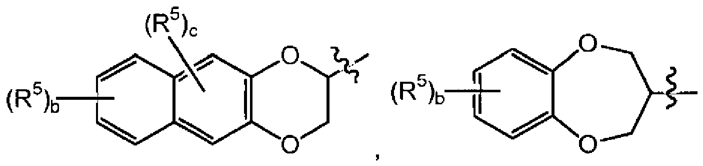 und 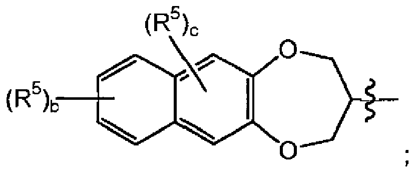
ausgewählt ist, wobei b für eine ganze Zahl von 0 bis 4 steht und c für eine ganze Zahl von 0 bis 2 steht;
R⁵ jeweils unabhängig voneinander aus der Gruppe bestehend aus Halogen und Niederalkyl ausgewählt ist;
mit der Maßgabe, dass dann, wenn 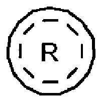 für 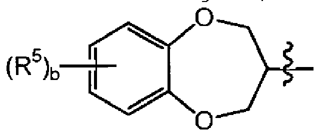 oder 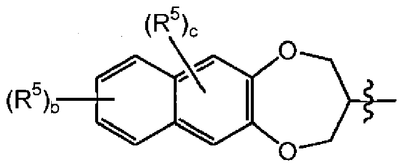 steht, a für 1 steht;
wobei "Nieder" bei Verwendung mit "Alkyl" eine Kohlenstoffkettenzusammensetzung mit 1-4 Kohlenstoffatomen bedeutet.

9. Verbindung oder pharmazeutisch unbedenkliches Salz davon zur Verwendung nach Anspruch 1 oder mindestens ein Anxiolytikum und eine Verbindung der Formel (I) oder ein pharmazeutisch unbedenkliches Salz davon als Kombinationspräparat zur Verwendung nach Anspruch 8, wobei die Verbindung der Formel (I) aus der Gruppe bestehend aus (2S)-(-)-N-(6-Chlor-2,3-dihydrobenzo[1,4]dioxin-2-yl-methyl)sulfamid und pharmazeutisch unbedenklichen Salzen davon ausgewählt ist.

10. Mindestens ein Anxiolytikum und eine Verbindung aus der Gruppe bestehend aus (2S)-(-)-N-(6-Chlor-2,3-dihydrobenzo[1,4]dioxin-2-ylmethyl)sulfamid und pharmazeutisch unbedenklichen Salzen davon als Kombinationspräparat für die gleichzeitige, getrennte oder aufeinanderfolgende Verwendung bei einer Methode zur Behandlung von Angst oder einer verwandten Störung, wobei die Angst oder verwandte Störung aus der Gruppe bestehend aus generalisierter Angstneurose, akuter Stresserkrankung, posttraumatischer Stresserkrankung, Zwangsneurose, sozialer Phobie (auch bekannt als soziale Angstneurose), spezifischer Phobie, Panikerkrankung ohne Agoraphobie, Agoraphobie ohne Vorgeschichte von Panikerkrankung und durch Substanzmissbrauch induzierter Angstneurose.

11. Verbindung oder pharmazeutisch unbedenkliches Salz davon zur Verwendung nach einem der Ansprüche 1-7 und 9 oder mindestens ein Anxiolytikum und eine Verbindung oder ein pharmazeutisch unbedenkliches Salz davon als Kombinationspräparat zur Verwendung nach Anspruch 8, 9 oder 10, wobei die Angst oder verwandte Störung aus der Gruppe bestehend aus generalisierter Angstneurose, akuter Stresserkrankung, posttraumatischer Stresserkrankung und Zwangsneurose ausgewählt ist.

12. Verbindung, pharmazeutisch unbedenkliches Salz davon zur Verwendung nach Anspruch 11 oder mindestens ein Anxiolytikum und eine Verbindung oder ein pharmazeutisch unbedenkliches Salz davon als Kombinationspräparat zur Verwendung nach Anspruch 11, wobei es sich bei der Angst oder verwandten Störung um generalisierte Angstneurose handelt.

13. Mindestens ein Anxiolytikum und eine Verbindung oder ein pharmazeutisch unbedenkliches Salz davon als Kombinationspräparat zur Verwendung nach Anspruch 8, 9 oder 10, wobei das Anxiolytikum aus der Gruppe bestehend aus Benzodiazepinen, selektiven Serotonin-Wiederaufnahmehemmmern, selektiven noradrenergen Wiederaufnahmehemmmern, Serotoninrezeptorantagonisten, Norepinephrin-Wiederaufnahmehemmmern, Dopamin-Wiederaufnahmehemmmern, Corticotropin-Releasing-Factor- Antagonisten, Monoaminoxidasehemmern, 5HT₁ₐ-Agonisten, Alpha2-adrenergen Antagonisten, GABA_{A}-Agonisten, GABA_{B}-Antagonisten, Cannabinoid-Agonisten, Neurokinin-Antagonisten, Kappa-Opioid-Antagonisten und Opioid-Receptor-Like-1-Agonisten ausgewählt ist.

14. Mindestens ein Anxiolytikum und eine Verbindung oder ein pharmazeutisch unbedenkliches Salz davon als Kombinationspräparat zur Verwendung nach Anspruch 13, wobei das Anxiolytikum aus der Gruppe bestehend aus Benzodiazepinen, selektiven Serotonin-Wiederaufnahmehemmmern (SSRIs) und selektiven noradrenergen Wiederaufnahmehemmmern ausgewählt ist.

15. Mindestens ein Anxiolytikum und eine Verbindung (1) oder ein pharmazeutisch unbedenkliches Salz davon als Kombinationspräparat zur Verwendung nach Anspruch 8, 9 oder 10, wobei das Anxiolytikum aus der Gruppe bestehend aus Diazepam, Flurazepam, Triazolam, Lorazepam, Alprazolam, Chlordiazepam, Oxazepam, Temazepam, Clonazepam, Buspiron, Fluoxetin, Sertralin, Paroxetin, Citalopram, Fluvoxamin, Nefazadon, Venlafaxin, Milnacipran, 3-(3-Amino-2-(R)-hydroxypropyl)-1-(4-fluorphenyl)-8-(8-methylnaphthalin-1-ylmethyl)-1,3,8-triazaspiro[4.5]decan-4-on, Carbamidsäure-2-(2-chlorphenyl)-2-(S)-hydroxyethylester und 1-[7-(4-Brom-2,6-dimethylphenyl)-2,5-dimethyl-7H-pyrrolo-[2,3-d]pyrimidin-4-yl]-4-piperidinmethanol ausgewählt ist.

16. Mindestens ein Anxiolytikum und eine Verbindung oder ein pharmazeutisch unbedenkliches Salz davon als Kombinationspräparat zur Verwendung nach Anspruch 15, wobei das Anxiolytikum aus der Gruppe bestehend aus Diazepam, Venlafaxin, Oxazepam und Pregabalin ausgewählt ist.

17. Mindestens ein Anxiolytikum und eine Verbindung der Formel (III) oder ein pharmazeutisch unbedenkliches Salz davon als Kombinationspräparat zur gleichzeitigen, getrennten oder aufeinanderfolgenden Verwendung bei einer Methode zur Behandlung von generalisierter Angstneurose, akuter Stresserkrankung, posttraumatischer Stresserkrankung, Zwangsneurose, sozialer Phobie (auch bekannt als soziale Angstneurose), spezifischer Phobie, Panikerkrankung mit oder ohne Agoraphobie, Agoraphobie ohne Vorgeschichte von Panikerkrankung und durch Substanzmissbrauch induzierter Angstneurose

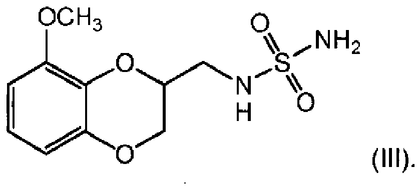

## Revendications

1. Composé de formule (I) ou l'un de ses sels de qualité pharmaceutique, pour emploi dans une méthode de traitement de l'anxiété ou d'un trouble apparenté, où l'anxiété ou le trouble apparenté sont choisis dans le groupe constitué par les suivants : trouble de l'anxiété généralisé, trouble de stress aigu, trouble de stress post-traumatique, trouble obsessionnel compulsif, phobie sociale (également appelée trouble d'anxiété sociale), phobie spécifique, trouble de panique avec et sans agoraphobie, agoraphobie sans antécédents de trouble de panique et trouble de l'anxiété induit par la toxicomanie 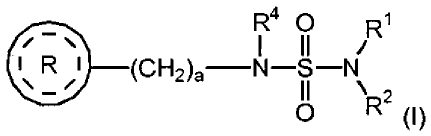 où
chacun des radicaux R¹ et R² est indépendamment choisi dans le groupe constitué par l'atome d'hydrogène et les groupements alkyle courts ;
R⁴ est choisi dans le groupe constitué par l'atome d'hydrogène et les groupements alkyle courts ;
a représente un entier compris entre 1 et 2 ; 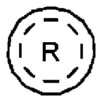 est choisi dans le groupe constitué par les groupements suivants : 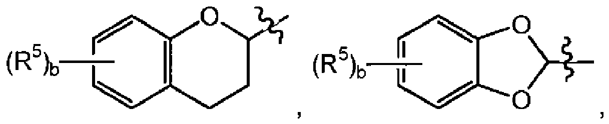 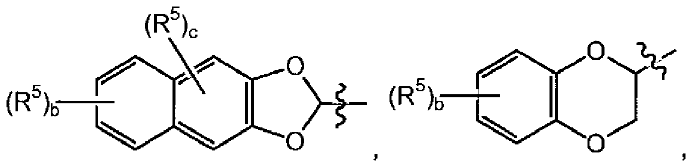 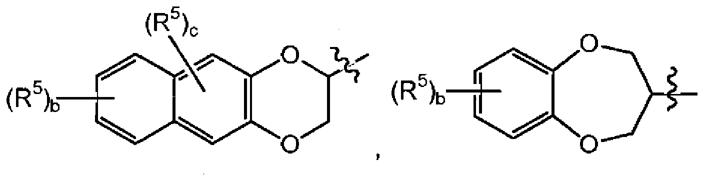 et 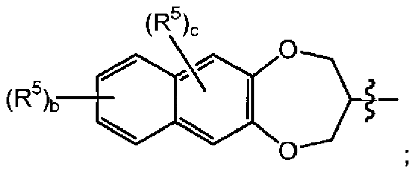
où b représente un entier compris entre 0 et 4 ; et où c représente un entier compris entre 0 et 2 ;
chacun des radicaux R⁵ est indépendamment choisi dans le groupe constitué par les atomes d'halogène et les groupements alkyle courts ;
à la condition que lorsque 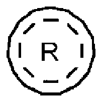 représente 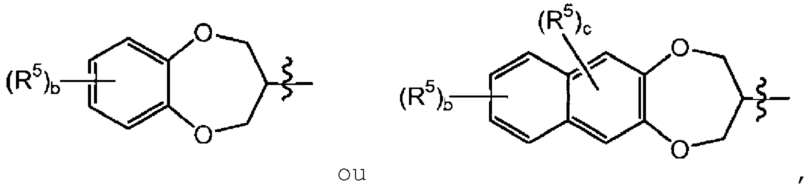 a est égal à 1 ;
le terme « court » dans le contexte des groupements « alkyle » désignant une chaîne carbonée composée de 1 à 4 atomes de carbone.

2. Composé ou l'un de ses sels de qualité pharmaceutique pour emploi conforme à la Revendication 1, où
chacun des radicaux R¹ et R² est indépendamment choisi dans le groupe constitué par l'atome d'hydrogène et les groupements alkyle courts ;
R⁴ est choisi dans le groupe constitué par l'atome d'hydrogène et les groupements alkyle courts ;
a représente un entier compris entre 1 et 2 ; 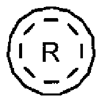 est choisi dans le groupe constitué par les groupements suivants : 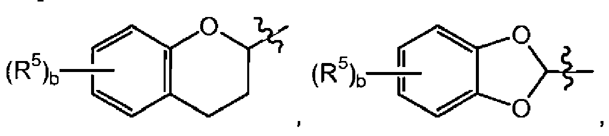 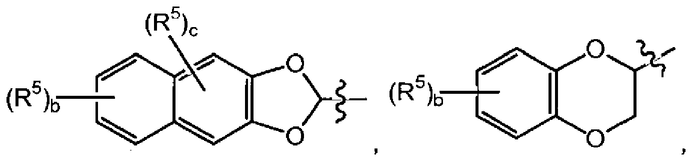 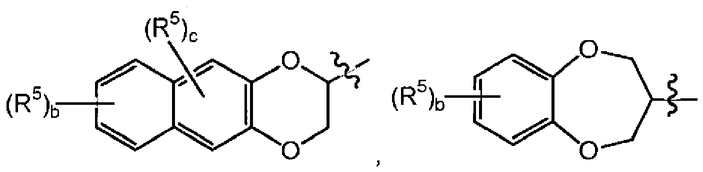 et 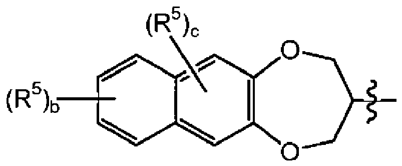 où b représente un entier compris entre 0 et 2 ; et où c représente un entier compris entre 0 et 1 ;
chacun des radicaux R⁵ est indépendamment choisi dans le groupe constitué par les atomes d'halogène et les groupements alkyle courts ;
à la condition que lorsque 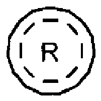 représente 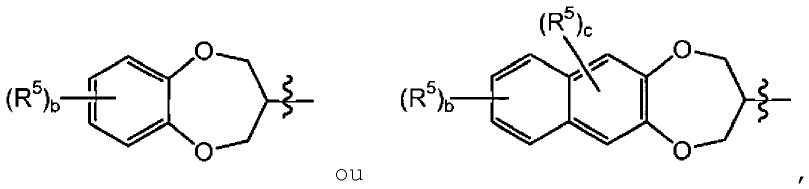 a est égal à 1.

3. Composé ou l'un de ses sels de qualité pharmaceutique pour emploi conforme à la Revendication 2, où
chacun des radicaux R¹ et R² est indépendamment choisi dans le groupe constitué par l'atome d'hydrogène et les groupements alkyle courts ;
R⁴ est choisi dans le groupe constitué par l'atome d'hydrogène et les groupements alkyle courts ;
a représente un entier compris entre 1 et 2 ; 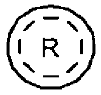 est choisi dans le groupe constitué par les groupements suivants : 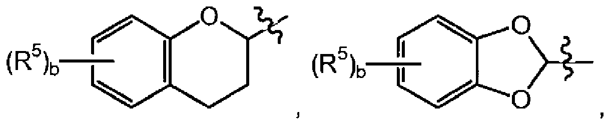 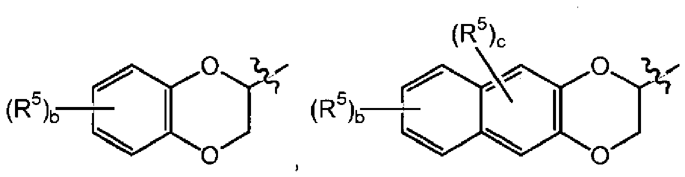 et 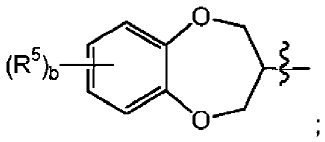 où b représente un entier compris entre 0 et 2 ; et où c est égal à 0 ;
chacun des radicaux R⁵ est indépendamment choisi dans le groupe constitué par les atomes d'halogène et les groupements alkyle courts ;
à la condition que lorsque 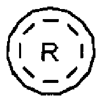 représente 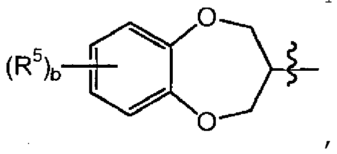 a est égal à 1.

4. Composé ou l'un de ses sels de qualité pharmaceutique pour emploi conforme à la Revendication 3, où
chacun des radicaux R¹ et R² est indépendamment choisi dans le groupe constitué par l'atome d'hydrogène et les groupements alkyle courts ;
R⁴ est choisi dans le groupe constitué par l'atome d'hydrogène et le groupement méthyle ;
a représente un entier compris entre 1 et 2 ; 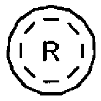 est choisi dans le groupe constitué par les groupements 2-(2,3-dihydro-benzo[1,4]dioxinyle), 2-(benzo[1,3]dioxolyle), 2-(3,4-dihydro-2H-benzo[1,4]dioxépinyle), 2-(6-chloro-2,3-dihydro-benzo[1,4]dioxinyle), 2-(6-fluoro-2,3-dihydro-benzo[1,4]dioxinyle), 2-(chromanyle), 2-(5-fluoro-2,3-dihydro-benzo[1,4]dioxinyle), 2-(7-chloro-2,3-dihydro-benzo[1,4]dioxinyle), 2-(6-chloro-benzo[1,3]dioxolyle), 2-(7-méthyl-2,3-dihydro-benzo[1,4]dioxinyle), 2-(5-chloro-2,3-dihydro-benzo[1,4]dioxinyle), 2-(6-bromo-2,3-dihydro-benzo[1,4]dioxinyle), 2-(6,7-dichloro-2,3-dihydro-benzo[1,4]dioxinyle), 2-(8-chloro-2,3-dihydro-benzo[1,4]dioxinyle), 2-(2,3-dihydro-naphto[2,3-b][1,4]dioxinyle) et 2-(4-méthyl-benzo[1,3]dioxolyle) ;
à la condition que lorsque 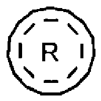 représente un groupement 2-(3,4-dihydro-2H-benzo[1,4]dioxépinyle), a est égal à 1.

5. Composé ou l'un de ses sels de qualité pharmaceutique pour emploi conforme à la Revendication 4, où
chacun des radicaux R¹ et R² est indépendamment choisi dans le groupe constitué par l'atome d'hydrogène et le groupement méthyle ;
R⁴ est choisi dans le groupe constitué par l'atome d'hydrogène et le groupement méthyle ;
a représente un entier compris entre 1 et 2 ; 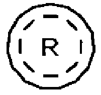 est choisi dans le groupe constitué par les groupements 2-(benzo[1,3]dioxolyle), 2-(2,3-dihydro-benzo[1,4]dioxinyle), 2-(6-chloro-2,3-dihydro-benzo[1,4]dioxinyle), 2-(7-chloro-2,3-dihydro-benzo[1,4]dioxinyle), 2-(7-méthyl-2,3-dihydro-benzo[1,4]dioxinyle), 2-(6-bromo-2,3-dihydro-benzo[1,4]dioxinyle) et 2-(6,7-dichloro-2,3-dihydro-benzo[1,4]dioxinyle).

6. Composé choisi dans le groupe constitué par le (2S)-(-)-N-(6-chloro-2,3-dihydro-benzo[1,4]dioxin-2-ylméthyl)-sulfamide ; et ses sels de qualité pharmaceutique pour emploi dans une méthode de traitement de l'anxiété ou d'un trouble apparenté, où l'anxiété ou le trouble apparenté sont choisis dans le groupe constitué par les suivants : trouble de l'anxiété généralisé, trouble de stress aigu, trouble de stress post-traumatique, trouble obsessionnel compulsif, phobie sociale (également appelée trouble d'anxiété sociale), phobie spécifique, trouble de panique avec et sans agoraphobie, agoraphobie sans antécédents de trouble de panique et trouble de l'anxiété induit par la toxicomanie.

7. Composé de formule (III) ou l'un de ses sels de qualité pharmaceutique, pour emploi dans le traitement de l'anxiété ou d'un trouble apparenté, où l'anxiété ou le trouble apparenté sont choisis dans le groupe constitué par les suivants : trouble de l'anxiété généralisé, trouble de stress aigu, trouble de stress post-traumatique, trouble obsessionnel compulsif, phobie sociale (également appelée trouble d'anxiété sociale), phobie spécifique, trouble de panique avec et sans agoraphobie, agoraphobie sans antécédents de trouble de panique et trouble de l'anxiété induit par la toxicomanie

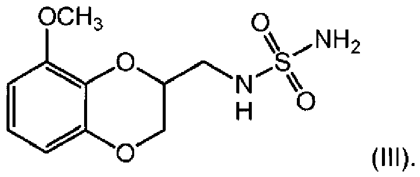

8. Au moins un anxiolytique et un composé de formule (I) ou l'un de ses sels de qualité pharmaceutique, sous forme d'une préparation combinée pour emploi simultané, séparé ou séquentiel dans une méthode de traitement de l'anxiété ou d'un trouble apparenté, où l'anxiété ou le trouble apparenté sont choisis dans le groupe constitué par les suivants : trouble de l'anxiété généralisé, trouble de stress aigu, trouble de stress post-traumatique, trouble obsessionnel compulsif, phobie sociale (également appelée trouble d'anxiété sociale), phobie spécifique, trouble de panique avec et sans agoraphobie, agoraphobie sans antécédents de trouble de panique et trouble de l'anxiété induit par la toxicomanie 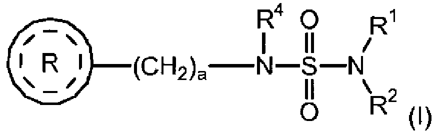 où
chacun des radicaux R¹ et R² est indépendamment choisi dans le groupe constitué par l'atome d'hydrogène et les groupements alkyle courts ;
R⁴ est choisi dans le groupe constitué par l'atome d'hydrogène et les groupements alkyle courts ;
a représente un entier compris entre 1 et 2 ; 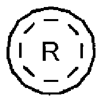 est choisi dans le groupe constitué par les groupements suivants : 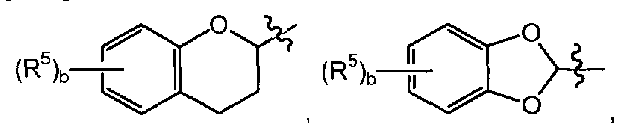 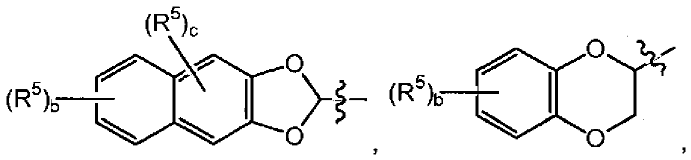 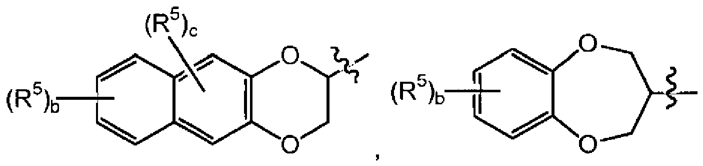 et 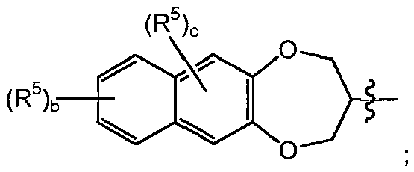
où b représente un entier compris entre 0 et 4 ; et où c représente un entier compris entre 0 et 2 ;
chacun des radicaux R⁵ est indépendamment choisi dans le groupe constitué par les atomes d'halogène et les groupements alkyle courts ;
à la condition que lorsque 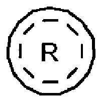 représente 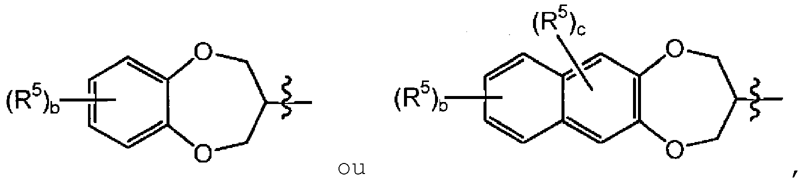 a est égal à 1 ;
le terme « court » dans le contexte des groupements « alkyle » désignant une chaîne carbonée composée de 1 à 4 atomes de carbone.

9. Composé ou l'un de ses sels de qualité pharmaceutique pour emploi conforme à la Revendication 1, ou le ou lesdits anxiolytiques et un composé de formule (I) ou l'un de ses sels de qualité pharmaceutique sous forme d'une préparation combinée pour emploi conforme à la revendication 8, où le composé de formule (I) est choisi dans le groupe constitué par le (2S)-(-)-N-(6-chloro-2,3-dihydro-benzo[1,4]dioxin-2-ylméthyl)-sulfamide ; ainsi que ses sels de qualité pharmaceutique.

10. Au moins un anxiolytique et un composé choisi dans le groupe constitué par le (2S)-(-)-N-(6-chloro-2,3-dihydro-benzo[1,4]dioxin-2-ylméthyl)-sulfamide ; et ses sels de qualité pharmaceutique sous forme d'une préparation combinée pour emploi simultané, séparé ou séquentiel dans une méthode de traitement de l'anxiété ou d'un trouble apparenté, où l'anxiété ou le trouble apparenté sont choisis dans le groupe constitué par les suivants : trouble de l'anxiété généralisé, trouble de stress aigu, trouble de stress post-traumatique, trouble obsessionnel compulsif, phobie sociale (également appelée trouble d'anxiété sociale), phobie spécifique, trouble de panique avec et sans agoraphobie, agoraphobie sans antécédents de trouble de panique et trouble de l'anxiété induit par la toxicomanie.

11. Composé ou l'un de ses sels de qualité pharmaceutique pour emploi conforme à l'une quelconque des Revendications 1 à 7 et 9, ou le ou lesdits anxiolytiques et un composé ou l'un de ses sels de qualité pharmaceutique sous forme d'une préparation combinée pour emploi conforme à la Revendication 8, 9 ou 10, où l'anxiété ou le trouble apparenté sont choisis dans le groupe constitué par les suivants : trouble de l'anxiété généralisé, trouble de stress aigu, trouble de stress post-traumatique et trouble obsessionnel compulsif.

12. Composé ou l'un de ses sels de qualité pharmaceutique pour emploi conforme à la Revendication 11, ou le ou lesdits anxiolytiques et un composé ou l'un de ses sels de qualité pharmaceutique sous forme d'une préparation combinée pour emploi conforme à la Revendication 11, où l'anxiété ou le trouble apparenté sont le trouble de l'anxiété généralisé.

13. Le ou lesdits anxiolytiques et un composé ou l'un de ses sels de qualité pharmaceutique sous forme d'une préparation combinée pour emploi conforme à la Revendication 8, 9 ou 10, où l'anxiolytique est choisi dans le groupe constitué par les suivants : benzodiazépines, inhibiteurs sélectifs de recapture de la sérotonine, inhibiteurs sélectifs de recapture des composés noradrénergiques, antagoniste des récepteurs de sérotonine, inhibiteurs de recapture de la norépinéphrine, inhibiteurs de recapture de la dopamine, antagoniste des récepteurs de la dopamine, antagonistes du facteur de libération de la corticotropine, inhibiteurs de monoamine oxydase, agonistes de 5HT₁ₐ, Alpha2-antagonistes des récepteurs adrénergiques, agonistes de GABA_{A}, agonistes de GABA_{B}, agonistes cannabinoïdes, antagonistes de neurokinine, antagonistes des récepteurs kappa-opioïdes et agonistes des récepteurs ORL1.

14. Le ou lesdits anxiolytiques et un composé ou l'un de ses sels de qualité pharmaceutique sous forme d'une préparation combinée pour emploi conforme à la Revendication 13, où l'anxiolytique est choisi dans le groupe constitué par les suivants : benzodiazépines, inhibiteurs sélectifs de recapture de la sérotonine (SSRI), et inhibiteurs sélectifs de recapture des composés noradrénergiques.

15. Le ou lesdits anxiolytiques et un composé de formule (I) ou l'un de ses sels de qualité pharmaceutique sous forme d'une préparation combinée pour emploi conforme à la Revendication 8, 9 ou 10, où l'anxiolytique est choisi dans le groupe constitué par les suivants : diazépam, flurazépam, triazolam, lorazépam, alprazolam, chlordiazépam, oxazépam, témazépam, clonazépam, buspirone, fluoxétine, sertraline, paroxétine, citalopram, fluvoxamine, néfazadone, venlafaxine, milnacipran, 3-(3-amino-2-(R)-hydroxy-propyl)-1-(4-fluoro-phényl)-8-(8-méthyl-naphtalén-1-ylméthyl)-1,3,8-triazaspiro[4.5]décan-4-one, carbamate de 2-(2-chloro-phényl)-2-(S)-hydroxy-éthyle et 1-[7-(4-bromo-2,6-diméthylphényl)-2,5-diméthyl-7H-pyrrolo[2,3-d]pyrimidin-4-yl]-4-pipéridineméthanol.

16. Le ou lesdits anxiolytiques et un composé ou l'un de ses sels de qualité pharmaceutique sous forme d'une préparation combinée pour emploi conforme à la Revendication 15, où l'anxiolytique est choisi dans le groupe constitué par les suivants : diazépam, venlafaxine, fluoxétine et prégabaline.

17. Au moins un anxiolytique et un composé de formule (III) ou l'un de ses sels de qualité pharmaceutique, sous forme d'une préparation combinée pour emploi simultané, séparé ou séquentiel dans une méthode de traitement des troubles suivants : trouble de l'anxiété généralisé, trouble de stress aigu, trouble de stress post-traumatique, trouble obsessionnel compulsif, phobie sociale (également appelée trouble d'anxiété sociale), phobie spécifique, trouble de panique avec et sans agoraphobie, agoraphobie sans antécédents de trouble de panique et trouble de l'anxiété induit par la toxicomanie
